# EUROPEAN PATENT SPECIFICATION

(11) **EP 4 415 617 B1**
(45) Date of publication and mention of the grant of the patent: **08.07.2026**
(21) Application number: 22802447.7
(22) Date of filing: 13.10.2022
(51) Int. Cl.: A61B 5/145, A61B 5/1468

(54) **SYSTEM FOR DISCARDING ERRONEOUS GLUCOSE MEASUREMENTS**
SYSTEM ZUR VERWERFUNG FEHLERHAFTER GLUKOSEMESSUNGEN
SYSTÈME DE REJET DES MESURES DE GLUCOSE ERRONÉES

(30) Priority: 14.10.2021 US 202163255903 P
(43) Date of publication of application: 21.08.2024
(73) Proprietor: Biolinq Incorporated, San Diego, CA 92121 (US)
(72) Inventor: YANG, Richard Chien, Carlsbad, CA 92009 (US); WINDMILLER, Joshua Ray, San Diego, CA 92130 (US)
(74) Representative: Cooley (UK) LLP
(86) International application number: PCT/US2022/078077
(87) International publication number: WO 2023/064877

(56) References cited:
- US-A1- 2008 189 051
- US-A1- 2014 005 505
- US-A1- 2016 310 049
- US-A1- 2017 000 391

## Description

### CROSS-REFERENCE TO RELATED APPLICATIONS

This application claims priority to U.S. Provisional Patent Application No. 63/255,903, filed October 14, 2021.

### BACKGROUND

Diabetes is a chronic disease in which the body does not produce or properly utilize insulin, a hormone that regulates blood glucose. Insulin or other oral therapies may be administered to an individual with diabetes to help regulate blood glucose levels, though blood glucose levels must nevertheless be carefully monitored to help ensure that timing and dosage are appropriate. In addition, management can be impacted by foods, activities, stress, sleep, and genetics. Without proper management of their condition, individuals with diabetes may suffer from a variety of complications resulting from hyperglycemia (high blood sugar levels) or hypoglycemia (low blood sugar levels).

Blood glucose monitors and continuous glucose monitors are devices that help individuals with diabetes manage their condition. Blood glucose monitors measure blood glucose levels from a sample of blood. For example, an individual with diabetes may obtain a blood sample through a fingerstick sampling mechanism, transfer the blood sample to a test strip with suitable reagents that react with the blood sample, and use a blood glucose monitor to analyze the test strip to measure glucose level in the blood sample. A continuous glucose monitor device includes electrochemical sensors that are used to continuously detect and quantify blood glucose levels by proxy measurement of glucose levels in interstitial fluid.

US2017/000391 describes a plurality of sensors configured to measure a physiological parameter.

### SUMMARY

Aspects of the current subject matter are directed to an ecosystem that enables the interconnection and data exchange between a blood glucose monitor and a continuous glucose monitor.

According to the invention, a system includes a microneedle array including a plurality of microneedles, where at least a first microneedle and a second microneedle of the plurality of microneedles are configured to sense glucose levels in dermal interstitial fluid of a user; and one or more processors and at least one memory storing instructions which, when executed by the one or more processors, result in operations including: determining that a first difference between a first glucose level measured by the first microneedle and a second glucose level measured by the second microneedle exceeds a first threshold; transmitting, to a blood glucose monitor, a request to receive a glucose measurement; determining, in response to the glucose measurement received from the blood glucose monitor, that a second difference between the first glucose level measured by the first microneedle and the glucose measurement from the blood glucose monitor exceeds a second threshold; and discarding, in response to determining that the second difference exceeds the second threshold, the first glucose level measured by the first microneedle in a resultant glucose level outputted on a user interface.

Further embodiments of the invention are set out in the appended claims.

Both the foregoing general description and the following detailed description are exemplary and explanatory only and are not restrictive. Further features and/or variations may be provided in addition to those set forth herein. For example, the implementations described herein may be directed to various combinations and sub-combinations of the disclosed features.

### DESCRIPTION OF THE DRAWINGS

The accompanying drawings, which are incorporated in and constitute a part of this specification, show certain aspects of the subject matter disclosed herein and, together with the description, help explain some of the principles associated with the disclosed implementations. In the drawings,
FIG. 1 depicts an illustrative schematic of a system that provides interconnection between a blood glucose monitor, a continuous glucose monitor, and other components according to aspects of the current subject matter;
FIG. 2 depicts an example of a data exchange sequence between a blood glucose monitor, a continuous glucose monitor, and one or more remote devices;
FIG. 3 depicts an example of a data exchange sequence directed to calibration and diagnostic aspects;
FIG. 4A, FIG. 4B, and FIG. 4C each depict an illustrative schematic of a device incorporating aspects of a blood glucose monitor and a continuous glucose monitor;
FIG. 5 depicts an illustrative schematic of a device incorporating aspects of a blood glucose monitor;
FIG. 6 depicts an illustrative schematic of a device incorporating aspects of a blood glucose monitor and a continuous glucose monitor;
FIG. 7 depicts an example schematic of a continuous analyte monitor that may be implemented with aspects of the current subject matter;
FIG. 8A depicts an illustrative schematic of a microneedle array. FIG. 8B depicts an illustrative schematic of a microneedle in the microneedle array depicted in FIG. 8A.
FIG. 9A depicts a cross-sectional side view of a columnar microneedle having a tapered distal end. FIGS. 9B and 9C are images depicting perspective and detailed views, respectively, of an embodiment of the microneedle shown in FIG. 9A.
FIG. 10 depicts an illustrative schematic of a columnar microneedle having a tapered distal end.
FIGS. 11A and 11B depict illustrative schematics of a microneedle array configuration. FIGS. 11C and 11D depict illustrative schematics of a microneedle array configuration.

When practical, similar reference numbers denote similar structures, features, or elements.

### DETAILED DESCRIPTION

Non-limiting examples of various aspects and implementations, as well as variations thereof, are described herein and illustrated in the accompanying drawings.

Aspects of the current subject matter are directed to an ecosystem that enables interconnection, data exchange, and shared capabilities between a blood glucose monitor and a continuous glucose monitor. In some variations, the blood glucose monitor and the continuous glucose monitor are connected such that data and/or instructions may be communicated between the blood glucose monitor and the continuous glucose monitor. The data and instructions may provide user-facing functionality or feedback to a user. For example, the data and instructions may be used to provide alerts and notifications to a user and to inform the user of one or more monitored or predicted data points. The interconnection between the blood glucose monitor and the continuous glucose monitor may also provide for improved functionality of either or both devices. For example, measurements may be used for calibration or verification processes.

Data streams from other data sources may be correlated or integrated with the data obtained by the blood glucose monitor and/or the continuous glucose monitor. The correlation or the integration of other data streams may be used to provide additional alerts or functionality to the user, as further described herein.

Aspects of the current subject matter also incorporate a network approach. Data and instructions from the blood glucose monitor, the continuous glucose monitor, and other user devices may be transmitted over a network and processed and/or stored by one or more remote processors, such as a smartphone, a tablet, a laptop, a smartwatch, a computer, a cloud server, and/or other processor-based devices. In some variations, an application software, such as a web-based application or mobile application or "app", may execute on a user device and be configured to receive information relating to operation of the blood glucose monitor, the continuous glucose monitor, and other user devices, to control operational aspects of the blood glucose monitor and the continuous glucose monitor, and to display information and provide for user input or control. Additional details are provided herein.

The interconnection and the data exchange between a blood glucose monitor and a continuous glucose monitor, as provided herein, provides many advantages to a user over traditional use of a blood glucose monitor and/or sporadic use of a continuous glucose monitor. For example, the traditional use of the blood glucose monitor on its own may provide little insight to the user. This may be especially true for a use case in which the user infrequently and/or randomly uses the blood glucose monitor. The user may notice a concerning glucose level, for example, a high glucose level, but may attribute the measurement as an outlier and therefore disregard the potential significance of the glucose level. In other instances, the user may be concerned with the glucose level but may not be aware of actions that may mitigate the concerning glucose level. The sporadic use of the continuous glucose monitor may similarly provide little insight to the user as to what actions are contributing to certain glucose levels and what actions to take. For example, in a use case in which the user sporadically uses the continuous glucose monitor, the user may not be able to identify any trends or patterns of the glucose levels and may accordingly not take appropriate corrective actions to mitigate concerning glucose levels when the continuous glucose monitor is not being used.

By combining aspects of the continuous glucose monitor with the blood glucose monitor, and by the optional incorporation of additional data streams, the user is presented with real-time, useful insights and guidance.

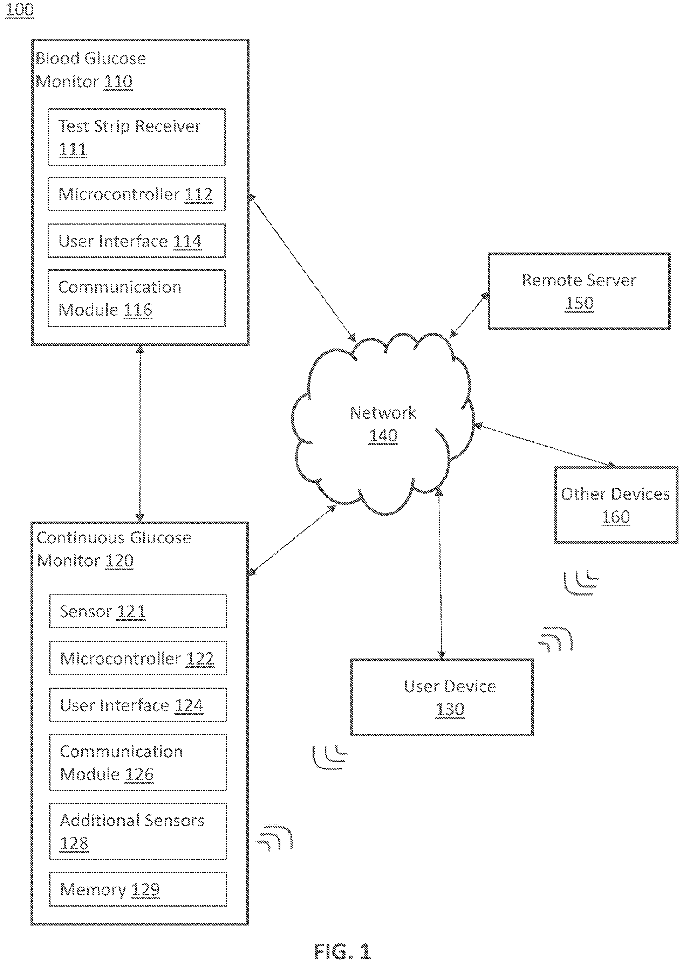

FIG. 1 depicts an illustrative schematic of a system 100 that provides interconnection, thereby enabling data exchange, between a blood glucose monitor 110 and a continuous glucose monitor 120, according to aspects of the current subject matter. A user device 130, a remote server 150, and other devices 160 are also interconnected to the blood glucose monitor 110 and the continuous glucose monitor 120. The connections may be through a network 140, or in some instances one or more of the components may communicate directly to each other.

The blood glucose monitor 110 is configured to measure blood glucose levels from a sample of blood. A user may obtain a blood sample through a fingerstick sampling mechanism and transfer the blood sample to a test strip with suitable reagents that react with the blood sample. A test strip receiver 111 receives the test strip with the blood sample for analysis. A microcontroller 112 of the blood glucose monitor 110 may include logic and modules configured to analyze the test strip with the blood sample to measure glucose level in the blood sample and output the glucose level on a user interface 114.

The blood glucose monitor 110 according to aspects of the current subject matter includes a communication module 116, such as a wireless communication module to communicate with one or more devices via wireless signals. For example, the communication module 116 may include a wireless transceiver configured to receive and transmit wireless signals. In some variations, the wireless transceiver is integrated into the microcontroller device 112. In some variations, the communication module 116 may communicate via a network 140 through, for example, Bluetooth, near field communication (NFC), Wi-Fi, cellular, radio-frequency identification (RFID), or any type of data transmission. The communication may use any of a plurality of communication standards, protocols, and technologies.

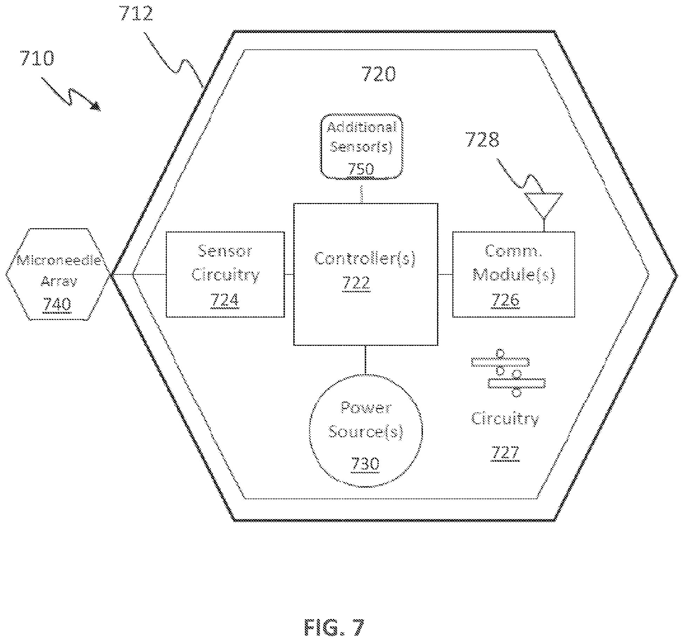

The continuous glucose monitor 120 is configured to monitor glucose levels of a user and includes a sensor 121 including one or more electrodes configured to perform electrochemical detection of glucose. For example, the sensor 121 may, in some variations, include a microneedle array including a plurality of microneedles that extend at least partially into the skin of a user. The continuous glucose monitor 120 includes a microcontroller 122 configured to perform analysis on sensor data from the sensor 121 and to output on a user interface 124 the sensor data, representations thereof, and other information. The continuous glucose monitor 120 includes a communication module 126, similar or equivalent to the communication module 116 of the blood glucose monitor 110. The communication module 126 is configured to communicate sensor data and/or instructions to other devices, such as the blood glucose monitor 110 and the user device 130. The continuous glucose monitor 120 may also include additional sensors 128, as further described herein. The continuous glucose monitor 120 may also include memory 129 configured to store the sensor data, additional sensor data, and program code and instructions. Additional aspects of an example continuous glucose monitor are provided below with respect to FIG. 7.

The user device 130, for example, a smartphone, a tablet, a laptop, a smartwatch, a computer, and/or the like, may include one or more processors executing a web-based application or a mobile application to handle sensor data, for example, displaying data, analyzing data, providing alerts, and providing notifications. The user device 130 may communicate with one or both of the blood glucose monitor 110 and the continuous glucose monitor 120. The user device 130 may also communicate with and receive information from the other devices 160, which may be used as additional data streams as further described herein. The other devices 160 may include a variety of devices that may be associated with a user, such as smart devices that monitor activity or health-related aspects of the user. Some examples include a smartwatch, a smart scale, and a smart medication dispenser.

As shown in FIG. 1, the network 140 interconnects the blood glucose monitor 110, the continuous glucose monitor 120, the user device 130, and the other devices 160. As described above, the network 140 may be a wireless network that provides for wireless data transmission between the interconnected devices. In some variations, the network 140 may include or be connected to a remote server 150 (e.g., a cloud-based server) for data analysis, transmission, and/or storage.

According to aspects of the current subject matter, sensor data representative of glucose levels of a user may be collected and used to provide prospective, real-time alerts to the user through the blood glucose monitor 110 or a connected device, such as the user device 130. Significantly, analysis of the sensor data may indicate certain patterns and trends of the glucose levels of the user. During or following use of the continuous glucose monitor 120, which may be used inconsistently and/or infrequently, the blood glucose monitor 110 may generate and output alerts based on instructions and analysis generated by the continuous glucose monitor 120. The alerts are intended to, in some variations, provide suggestions on actions that, if taken, may be beneficial to the user. For example, at times at which the user typically has a high glucose level, the alert may provide an indication to fingerstick and test the blood glucose level. The alert may also provide a corrective action, such as waiting to eat, eating a healthy meal, or taking a walk.

Moreover, additional data streams may be incorporated with the sensor data to provide further insights. As an example, accelerometer data is correlated with a level of activity of the user. If the user is sedentary at the time at which the user typically has a high glucose level, the alert may suggest that the user engage in physical activity. In other cases, the accelerometer data is used to provide insulin absorption estimates in conjunction with the sensor data, and recommendations to reduce subsequent insulin doses are made. Accelerometer data may also be utilized to estimate pharmacotherapy adherence, and patterns of activity and inactivity are correlated with overall glycemic profiles and provide reminders for pharmacotherapy treatment.

In some variations, as further described herein, a mobile or web-based application may be implemented to generate the alerts, provide related information and suggestions, and allow for user input and control. The application may further support various health-related goals with the incorporation of the sensor data from the continuous glucose monitor 120 and the incorporation of blood glucose levels from the blood glucose monitor 110. Additional data streams may be incorporated to provide more data that may be used to further refine the alerts generated by the blood glucose monitor 110.

According to aspects of the current subject matter, the continuous glucose monitor 120 may create a baseline representation of glucose levels based on sensor data obtained from the sensor 121. For example, sensor data representative of a glucose level of the user is continuously obtained by the sensor 121, and the microcontroller 122 receives and analyzes the sensor data to create the baseline representation of the glucose levels. The baseline representation is a record or a representation of the glucose levels versus time. In some variations, a timestamp may be associated with each measured glucose level such that the baseline representation provides an indication of the measured glucose level at a particular time for the duration of use, or a portion of the duration of use, of the continuous glucose monitor 120. In some variations, the baseline representation is based on glucose levels obtained at discrete times.

In some variations, the record or the representation may be raw data from the sensor 121. In some variations, the microcontroller 122 may process the raw data from the sensor 121 according to various algorithms. For example, the raw data may be processed to convert the sensor data to glucose levels represented in values of mg/dL. For example, the sensor data obtained from the sensor 121 including the microneedle array may have a direct correlation with blood glucose levels, and accordingly the sensor data may be converted to glucose levels represented in values of mg/dL. Other units may be used in accordance with the current subject matter. Other processing of the sensor data may be implemented by the microcontroller 122. For example, various types of averaging, filtering, and signal processing may be implemented.

The baseline representation may be associated with the user and stored in memory, for example, the memory 129 of the continuous glucose monitor 120 or memory associated with or contained within the remote server 150.

The continuous glucose monitor 120, or alternatively the remote server 150, may analyze the baseline representation to identify one or more parameters and/or characteristics of the baseline representation. In some variations, the one or more parameters and/or characteristics are characterized as belonging to a defined category of data relationships. For example, different types of data patterns, trends, and/or characteristics may be defined, and the baseline representation may be analyzed to identify one of the types of data patterns, trends, and/or characteristics, which may be used to generate notifications and/or recommendations. The characteristics may include, for example, time in range and data anomalies. Time in range may be defined as an amount of time that glucose levels are between a lower limit and an upper limit, both of which may be defined values that may be adjustable. For example, the lower limit and/or the upper limit may be user-defined and/or user-adjustable, device-defined, or system-defined. A data anomaly may be defined as a glucose level that exceeds a predefined rate of change that is not, in some variations, physiologically possible. As another example, mealtimes may be identified based upon a rate of change in measured glucose levels. For example, a rate in change of glucose levels equal to or exceeding about 2 mg/dL/min may be representative of a meal excursion. In some instances, a rate in change of glucose levels less than about 1 mg/dL/min may be representative of normal physiologic fluctuations.

Recommendations on future mealtimes may be provided based upon the baseline representation where the measured glucose level is typically low. Sleep patterns may also be identified from the baseline representation, and recommendations related to sleep may be provided. The baseline representation may also indicate differences between days of the week and weekdays and weekends. For insulin-taking users, the baseline representation may also provide an insight into missed insulin medications, and reminders related to medication may be provided.

In some variations, a pattern is defined as a set of data that follows a recognizable form. In the context of the continuous glucose monitor 120, one example of a pattern is a repeated occurrence of a spike in glucose levels at or near the same time each day. In some variations, a pattern definition may be defined and may indicate parameters or characteristics of the data set included in the baseline representation that qualify a portion of the data set as a pattern. The microcontroller 122 refers to the pattern definition when analyzing the baseline representation to identify patterns. In some variations, the data may need to follow a recognizable form at least, for example, two or three times to qualify as a pattern. In some variations, the pattern definition is established by a manufacturer and applied by the microcontroller 122. In some variations, the pattern definition may be modified by the user or an administrator through input to the user device 130 connected to the continuous glucose monitor 120. For example, the user may enter and/or modify parameters related to operation of the continuous glucose monitor 120 via a mobile or web-based application.

In some variations, a trend is defined as a general direction of data over a time period. In the context of the continuous glucose monitor 120, one example of a trend is glucose levels increasing in an upward direction between certain times of a day. Various parameters or characteristics that define what constitutes a trend in the data set of the baseline representation may be established as a trend definition, which may be set by a manufacturer and applied by the microcontroller 122. The trend definition may be modified by the user. The microcontroller 122 refers to the trend definition when analyzing the baseline representation to identify trends.

The microcontroller 122 or the remote server 150 may analyze the baseline representation, the result of which is the identification of one or more patterns, trends, and other characteristics. In addition to the pattern definition and the trend definition, the microcontroller 122 or the remote server 150 may utilize a machine learning algorithm to identify the patterns and the trends, as well as other characteristics of the baseline representation. In some variations, the patterns and the trends are updated as more data is added to the baseline representation.

For each of the one or more patterns, trends, and/or other characteristics that are identified from the baseline representation, corresponding notifications may be defined. For example, the continuous glucose monitor 120 may utilize a notification definition to assign notifications to the patterns, trends, and other characteristics. The notification definition defines the type of notification to be generated based on the type of pattern, trend, and other characteristics. The notification definition may vary based on a variety of factors, including but not limited to number of days of use of the continuous glucose monitor 120, number of elapsed days since identification of the pattern, the trend, or the other characteristic, severity of the baseline representation associated with the pattern, the trend, or the other characteristic, and number of days following end of use of the continuous glucose monitor 120. Examples of the notifications that may be generated include a visual indicator such as a flashing light emitting diode (LED) and/or a textual display on the user interface, an audible indicator such as an emission of an audio tone and/or a spoken message, and a haptic indicator such as haptic feedback and/or vibration. In the implementation of a mobile application, the notifications may be generated and accessible to the user via the user device 130. The notification definition may be user-defined and/or user-adjustable, device-defined, and/or system-defined. The notification definition may be stored locally on the continuous glucose monitor 120 or at the remote server 150, the user device 130, and/or another device connected to the network 140.

The continuous glucose monitor 120 may, upon identification of the pattern, the trend, or the other characteristic, transmit an instruction to the blood glucose monitor 110 to output the associated notification. The notification may be sent in an instruction signal to the blood glucose monitor 110. Upon receipt of the instruction signal containing the notification, the blood glucose monitor 110 generates the notification for the user's attention. The instruction signal may be sent at or near a time at which the notification should be generated, or the continuous glucose monitor 120 may send the instruction signal in advance, indicating in the instruction signal a time at which the notification should be generated.

As an example, the baseline representation of the glucose levels of a user may indicate that the user's glucose level is consistently high (as defined by an upper threshold value adjusted or set by the user) at 3:00 PM or within a defined range of 3:00PM. A notification that suggests the user test his or her glucose level (or take other action) at 3:00 PM may be generated by the blood glucose monitor 110. The notification may be generated one or more times before 3:00 PM and again at 3:00 PM. In some variations, varying frequencies and intensities of the notification may be used. The notification may include an icon and/or a textual message that may indicate the one or more suggested next steps, for example, stop eating, postpone next meal, enjoy a lighter meal, and/or check your glucose level. The suggested next steps may be selected based upon the particular pattern, trend, and/or other characteristic that is identified. For example, in some variations, one or more next steps may be defined and associated with one or more patterns, trends, or other characteristics, and this information may be accessible to the blood glucose monitor 110, the continuous glucose monitor 120, and/or any of the other devices connected to the network 140.

In some variations, the pattern, the trend, or the characteristic is sent from the continuous glucose monitor 120 to the blood glucose monitor 110, and the blood glucose monitor 110 determines an output that should be generated based on the pattern, the trend, or the characteristic (rather than the continuous glucose monitor 120 determining the output). In some variations, a remote device such as the user device 130 or the remote server 150 implements the analysis of the baseline representation and/or the determination of the notification to generate. In some variations, the notification is generated and displayed via the application executing on the user device 130.

In addition to generating notifications based on actions to take, notifications of positive (e.g., encouraging) reinforcement may also be generated. Sensor data from two or more predefined durations of time may be compared to identify changes indicative of improved glucose levels. For example, sensor data from 8:00 AM to noon from a current day may be compared to sensor data from 8:00 AM to noon from a preceding day. If the glucose levels of the current day are lower on average than those from the preceding day without falling below a threshold level, the continuous glucose monitor 120 may generate and transmit an instruction to the blood glucose monitor 110 to output a notification indicating the improved glucose levels. In some variations, sensor data from one day is compared against previous days to identify one or more changes indicative of improved measurements or statistics. A corresponding notification is outputted by the blood glucose monitor 110 at a defined time, such as the morning of the following day to alert the user to the improvements made the previous day. In some variations, portions of sensor data from a particular day are compared against corresponding portions of sensor data from preceding days until one or more improved measurements or statistics are identified. The portions of the sensor data may be adjusted until one or more improved measurements or statistics are identified. The time at which the notification is generated may be established and defined by the user through, for example, use of the user device 130 on which a mobile application is executing. An improved measurement or statistic may in some variations be defined as a glucose level at a particular time or over a period of time being lower on average than a previous glucose level at a corresponding time or period without falling below a threshold level.

In some variations, the blood glucose monitor 110, the user device 130, and/or the remote server 150 may store the baseline representation, the patterns, the trends, and the other characteristics and may continue to use the patterns, the trends, and the other characteristics after the user has stopped use of the continuous glucose monitor 120. The continued use of the baseline representation analysis may be based on one or more criteria, such as number of days of sensor data, amount of time since last update to sensor data, and amount of variation between sensor data (e.g., if sensor data is consistent day to day). As time since use of the continuous glucose monitor 120 increases, the number of days of sensor data for use in generating outputs may decrease (e.g., after one day of non-use, use seven most recent days' worth of data; after two days of non-use, use six most recent days' worth of data; etc.). The criteria to determine the continued use of the baseline representation analysis may be user-defined and/or adjustable, device-defined, and/or system-defined. For example, a user may define and/or adjust through settings and controls on a mobile application. In some variations, parameters related to the continued use of the baseline representation may initially be defined and established by one or more of the blood glucose monitor 110, the user device 130, and the remote server 150. In some variations, the user may adjust the initially-set parameters. In some variations, the user is not permitted to adjust the initially-set parameters. In some variations, an authentication process (e.g., inputting of a password or the like) is required for the adjustment of the initially-set parameters.

The sensor data and the measurements from the blood glucose monitor 110 may be correlated at similar times (e.g., same or similar times of day) to identify if suggested or other behavioral changes are being applied by the user or if behavioral changes are working or having an impact on the user. Similar times may be defined as a time that is within +/- 1% to 10% of the time that the measurements are being correlated with. In some variations, a correlation time for correlating the sensor data and the measurements from the blood glucose monitor 110 may be user-defined and/or adjustable, device-defined, and/or system-defined. For example, each fingerstick measurement may be date and time stamped, and an estimate of a match may be determined. If there is improvement (e.g., the blood glucose level is now lower in the morning without going below a lower threshold), this may be an indication that adjustments were made by the user and are helpful. If there is not an improvement (e.g., the blood glucose level is not lower or is higher), a recommendation may be made to incorporate adjustments and/or begin another session of use of the continuous glucose monitor 120 to obtain continuous, real-time feedback. In some instances, if there is not an improvement, a recommendation may be made to take additional fingerstick measurements to determine if a pattern exists. If the pattern from the blood glucose monitor 110 is different from the baseline representation without significant changes made to activity and/or medication, a recommendation may be made to begin another session of use of the continuous glucose monitor 120. In some variations, the baseline representation may be newly generated through use of a new continuous glucose monitor 120 and compared to the previous baseline representation to be used to identify effectiveness of therapies, treatments, and/or recommendations. In some variations, the new baseline representation may be generated through a series of blood glucose measurements from the blood glucose monitor 110. By analyzing the differences between two baseline representation curves over an equivalent period of time, adjustments in terms of medication timing, meal timing, activity timing, and/or the like may be identified. Additionally, by providing a depiction of the baseline representations to the user, the user may gain additional understanding and insights as to how behavior and treatments affect glucose levels. A depiction of the baseline representations may be presented to the user through the app on the user device 130, for example.

If a new continuous glucose monitor 120 is linked with the user and/or the blood glucose monitor 110, the analysis algorithm may adapt the new sensor data with the old sensor data based on the amount of time between use of the old continuous glucose monitor 120 and the new continuous glucose monitor 120, user selection, and other criteria. If a predetermined amount of time (e.g., six months) has elapsed between use of the old continuous glucose monitor 120 and the new continuous glucose monitor 120, the old sensor data may be discarded. The predetermined amount of time may be defined and/or adjusted. For example, the predetermined amount of time may be user-defined and/or adjustable, device-defined, and/or system-defined. In some variations, prior to discarding the old sensor data, an analysis may be done to determine a difference between the new sensor data and the old sensor data. If the difference meets or exceeds a threshold level, the old sensor data is discarded. In some variations, a portion of the old sensor data may be more relevant than other portions of the old sensor data, and the more relevant portion may be integrated with the new sensor data while the less relevant portion is discarded. Sensor data may be defined as relevant by threshold levels. For example, a first portion of the old sensor data may be below the threshold level and is thus relevant, while a second portion of the old sensor data may be above the threshold level and is thus defined as not relevant.

In some variations, data streams from other data sources may be correlated or integrated with the baseline representation. For example, accelerometer data, other activity data, light sensor data, sleep data, temperature data, heart rate information, global positioning system (GPS) data, lactate measurements, ketone measurements, cortisol measurements, and other analyte measurements may be used. The data streams may come from the continuous glucose monitor 120, such as from the additional sensors 128. For example, the continuous glucose monitor 120 may include an accelerometer, an ambient light sensor, a temperature sensor, and additional sensors to measure and quantify analytes in addition to glucose, for example, cortisol. The data streams may come from other sources, such as the other devices 160 connected to the network 140 and/or the user device 130. For example, data from the user device 130 and/or another device such as a wearable worn by the user may be used.

Data streams from other data sources may be correlated or integrated with the data obtained by the blood glucose monitor 110 and/or the continuous glucose monitor 120. The correlation or the integration of other data streams may be used to provide additional notifications or functionality to the user.

For example, the accelerometer data indicates activity level of the user and can be used to provide additional insight to the user regarding how the activity level correlates with the glucose level. For example, in a scenario in which the user's glucose level is consistently high at 3:00 PM, if the activity level is consistently low at 3:00 PM, the notification to be generated by the blood glucose monitor 110 may suggest increased activity. If, on the other hand, the activity level is quantified as moderate to high at 3:00 PM, the analysis may indicate that caloric intake is a more significant factor, and the generated notification may accordingly provide a suggestion related to eating habits earlier in the day. Activity level may be defined and quantified based on various parameters, such as number of steps, heart rate, oxygenation level, and the like. Thresholds for the number of steps within a given time period, heart rate, and/or oxygenation level may be defined and correlated with a particular activity level.

Activity data from other apps may be also used to identify exercise and activity impacts on glucose level measurements. For example, the baseline representation may be augmented with workout and activity information, allowing for the user to directly see the glucose level impact of various workouts and activities. In some variations, recommendations may be provided to suggest workouts or activities, including duration and intensity levels, to more positively impact glucose levels.

GPS data from a user device 130 or other device 160 may be used to identify the effects of environmental factors, such as being at work, commuting, or visiting friends or family, on glucose level measurements. By identifying locations that negatively affect glucose level measurements, notifications that recommend actions that positively impact glucose level measurements may be provided to the user.

In other examples, sleep data from a sleep monitor, heart rate information from a wearable, and/or data from a smart scale may be integrated or augmented into the baseline representation to provide for the user to see benefits and effects on glucose levels. Associated recommendations may be provided. Data from a smart scale may include weight, lean body mass, body fat mass, muscle mass, and/or basal metabolic rate (BMR), each of which may provide additional insight to the user.

The incorporation or augmentation of data streams from other data sources with the baseline representation of the glucose levels may be used to create health-related goals. A mobile or web-based application may be accessed by the user for selecting or inputting goals, such as those related to exercise and weight loss. A baseline representation including a representation of glucose levels measured by the continuous glucose monitor 120 and a representation of one or more of activity level, sleep patterns, and caloric intake may be generated to be used as a comparison point against future data. The activity level, the sleep patterns, and the caloric intake may be obtained from one or more other sensors, such as the additional sensors 128, the user device 130, other apps, or other devices, or may be inputted by the user. For example, the user may enter details related to amount of sleep, activity, and food consumed. In some variations, one or more tags, codes, or the like associated with one or more meals may be scanned by the mobile application to obtain related caloric data.

The user device 130 and/or the remote server 150 may store the baseline representation and continue to gather data including glucose levels, activity, sleep, and/or caloric intake. The collected data may be monitored and compared against the baseline representation, from which patterns, trends, characteristics, and changes may be identified. Current glucose level measurements may be obtained from the blood glucose monitor 110, which may output a notification to the user take a fingerstick at predetermined times. The user device 130 and/or the remote server 150 may send instructions to the blood glucose monitor 110 on when to output notifications related to when a fingerstick measurement should be taken. Notifications indicative of changes and including recommendations may be provided to the user via the mobile application. The notifications may encourage the user to get more exercise, get more sleep, destress, eat at different times, and/or provide positive reinforcement and encouragement. The notifications related to when to take a fingerstick measurement may be determined based on one or more defined thresholds compared against one or more corresponding data points of the collected data.

While implementations described herein may be directed to the blood glucose monitor 110 outputting the notifications, the notifications may additionally or alternatively be provided by the user device 130 or the other devices 160. For example, the notifications may be provided through a mobile or web-based app or through a message, such as a text message or email message generated and sent to the user. As one example, a compilation of notifications may be created and sent daily to the user. As another example, the user may access a secure website, access to which provides the notifications.

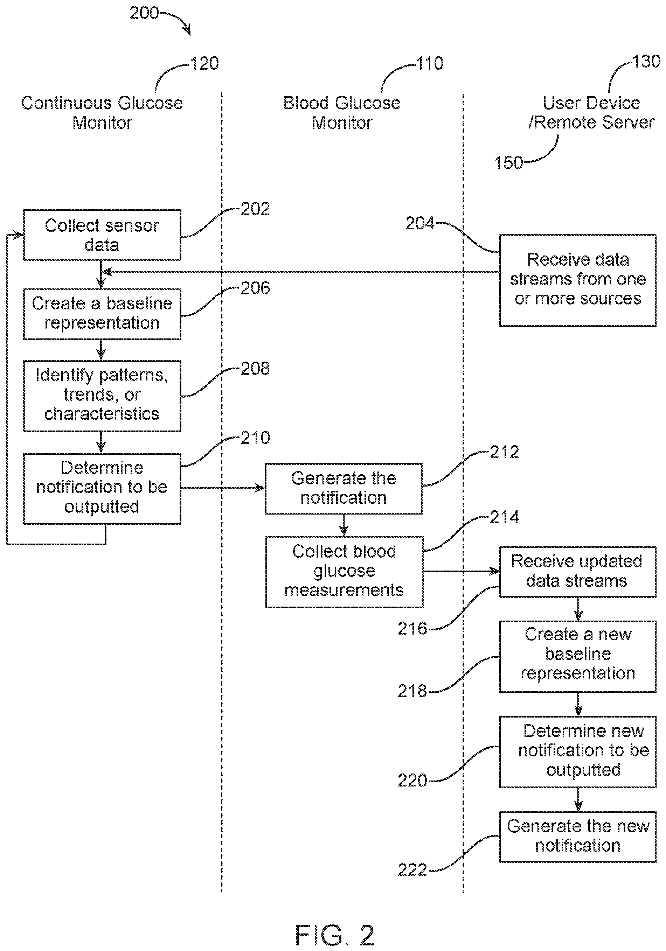

FIG. 2 depicts an example of a data exchange sequence 200 between the blood glucose monitor 110, the continuous glucose monitor 120, and one or more remote devices, such as the user device 130 and/or the remote server 150.

At 202, the continuous glucose monitor 120 collects sensor data representative of glucose levels of a user. For example, the continuous glucose monitor 120 is configured to monitor glucose levels of a user and includes the sensor 121 including one or more electrodes configured to perform electrochemical detection of glucose. In some variations, the sensor 121 includes a microneedle array including a plurality of microneedles that extend at least partially into the skin of a user. The microcontroller 122 performs analysis on sensor data from the sensor 121.

At 204, the user device 130 and/or the remote server 150 receive data streams representative of other types of data that may impact glucose levels of the user. The data streams may be generated concurrent with the collection of sensor data and may be provided to the continuous glucose monitor 120.

At 206, a baseline representation is created. The baseline representation is based on the sensor data obtained from the sensor 121. For example, sensor data representative of a glucose level of the user is continuously obtained by the sensor 121, and the microcontroller 122 receives and analyzes the sensor data to create the baseline representation of the glucose levels. The baseline representation is a record or a representation of the glucose levels versus time. In some variations, the baseline representation is augmented with the data from the data streams. In some variations, the data from the data streams is integrated into the baseline representation. In yet other variations, the initial baseline representation includes only the glucose level measurements.

At 208, the baseline representation of glucose levels for the user, which represents the measured glucose levels during use of the continuous glucose monitor 120, is analyzed to identify one or more patterns, trends, and other characteristics, which may be used to generate notifications and/or recommendations.

At 210, a notification to be outputted is determined. The notification is based on the analysis of the baseline representation and more particularly based on the identified pattern, trend, and/or other characteristic.

At 212, the blood glucose monitor 110 generates the notification. The notification may be generated in response to receiving an instruction or signal from the continuous glucose monitor 120. The instruction or the signal may indicate parameters of the notification to be generated, for example, when to generate the notification and what type of notification to generate.

The steps 202 through 212 may be repeated one or more times. In some variations, any number of notifications may be generated. In some variations, limits are set as to the number and/or frequency of notifications. In some variations, notifications related to a pattern, trend, or characteristic that meet or exceed a threshold are generated.

At 214, the blood glucose monitor 110 may determine blood glucose measurements based on one or more blood samples from the user. The blood glucose measurements may be taken in response to one or more notifications from the continuous glucose monitor 120. In some variations, the blood glucose measurements are used to identify changes to the baseline representation created at 206 based on the sensor data from the continuous glucose monitor 110.

At 216, the user device 130 and/or the remote server 150 receive updated data streams from other data sources, as further described herein. The updated data streams may be related to sleep, activity, location, temperature, meals, and various health-related aspects.

At 218, the user device 130 and/or the remote server 150 may create a new baseline representation based off of the blood glucose measurements from the blood glucose monitor 110 and the updated data streams. The new baseline representation may be used as a comparison with the baseline representation to identify impact on glucose levels.

At 220, a notification to be outputted is determined. The notification is based on the new baseline representation and/or the comparison of the new baseline representation with the baseline representation.

At 222, the notification is outputted. For example, the notification is generated and accessible on an app executing on the user device 130. In some variations, the notification is stored and compiled with other notifications.

In additional variations, blood glucose level measurements collected by the blood glucose monitor 110 may be used in calibration and diagnostic processes. For example, the blood glucose level measurements may be used as a retrospective snapshot or tool to adapt an algorithm used by the continuous glucose monitor 120 in the measurement of glucose levels. For example, the blood glucose level measurements from the blood glucose monitor 110 may indicate that the measurements from the continuous glucose monitor 120 are off in a general direction. For example, the continuous glucose monitor 120 may be generating measurements that are generally higher or generally lower than those from the blood glucose monitor 110. With this knowledge obtained from historical data from the blood glucose monitor 110, the algorithm implemented by the continuous glucose monitor 120 may be adapted to account for the measurement discrepancies.

In some variations, the blood glucose monitor 110 may, with providing the glucose level measurements, wake the continuous glucose monitor 120 to establish date and time parameters and to indicate that synchronization of data is required. The synchronization may include applying the measurements from the blood glucose monitor 110 to corresponding ones of the measurements from the continuous glucose monitor 120 to determine how the measurements differ. Upon determination of such a difference characteristic, or function, the difference characteristic may be applied to future measurements made by the continuous glucose monitor 120.

In some variations, the difference characteristic may be applied to a plurality of other continuous glucose monitors. For example, the difference characteristic may be uploaded to the remote server 150, from which the plurality of other continuous glucose monitors may access the difference characteristic.

In some variations, a plurality of difference characteristics from corresponding ones of a plurality of blood glucose monitors may be analyzed to determine one or more trends or commonalities between the difference characteristics. The plurality of difference characteristics may be merged or combined to create a compiled difference characteristic, which may be applied through the remote server 150 and the network 140 to a plurality of continuous glucose monitors. In some implementations, an algorithm operating on each of the plurality of continuous glucose monitors may be updated to account for the compiled difference characteristic.

In some variations, glucose level measurements of a particular user as determined by the continuous glucose monitor 120 may tend to be offset from the measurements obtained by the blood glucose monitor 110. For example, the continuous glucose monitor 120 on the particular user may tend to produce measurements low compared to the measurements from the blood glucose monitor 110. A difference characteristic for the particular user may be determined and may be applied to the algorithm implemented by the continuous glucose monitor 120. When the particular user begins using a new continuous glucose monitor 120, the previously determined difference characteristic specific to the particular user may then be applied to the algorithm. In some variations, the difference characteristic associated with the particular user may be stored in the user device 130 and/or the remote server 150 and then applied to the new continuous glucose monitor 120 upon application.

The glucose level measurements from the blood glucose monitor 110 may also be used as a diagnostic tool for the continuous glucose monitor 120. In some variations, glucose level measurements may be made from one or more of a plurality of microneedles of the microneedle array of the continuous glucose monitor 120. For example, one or more of the plurality of microneedles may generate sensor data indicative of glucose level measurements. In some instances, a discrepancy between the glucose level measurements from the microneedles may exist. In some instances, some discrepancy may be expected. In other instances, a discrepancy may indicate a potential problem with, for example, the continuous glucose monitor 120. For example, a discrepancy or difference between measurements of about 20 mg/dL or less may be deemed as acceptable for values less than 70 mg/dL, and a discrepancy or difference between measurement of about 20% or less for values equal to or exceeding 70 mg/dL may be deemed as acceptable. Discrepancies or differences exceeding these values may serve as an indication that there is a potential problem with one or more of the measurements.

The continuous glucose monitor 120 monitors the glucose level measurements from each of the plurality of microneedles that is generating sensor data. Upon a determination that a difference equal to or exceeding a first threshold exits between two or more of the microneedles, the continuous glucose monitor 120 may generate and transmit a signal to the blood glucose monitor 110 and/or to the user device 130 (or to another device connected to the network 140) requesting an output be generated that requests the user take a fingerstick measurement. The blood glucose monitor 110 then transmits the glucose level measurement to the continuous glucose monitor 120, which may compare the received value to the glucose level measurements from each of the plurality of microneedles generating sensor data. The glucose level measurements from the microneedles that differ by at least a second threshold from the glucose level measurement from the blood glucose monitor 110 may be identified as potentially erroneous and discarded. For example, the measurement from the identified microneedle is not used in a resultant glucose level that is displayed to the user. In some variations, the first threshold and the second threshold may be equal. In some variations, the first threshold differs from the second threshold. In some variations, the first threshold is less than (e.g., more sensitive than) the second threshold. In some variations, the microneedle identified as producing erroneous measurements may continue to be monitored. When the difference measurements with the identified microneedle no longer equal or exceed the first threshold or the second threshold, the measurements from the identified microneedle may then be incorporated in the resultant measurement determined by the continuous glucose monitor 120. In some variations, measurements from the identified microneedle are monitored from a threshold period of time. After the threshold period of time has elapsed and if the difference measurements continues to exceed the first threshold or the second threshold, the identified microneedle is no longer used. In some variations, if one or more microneedles are not in use due to the difference measurements exceeding the first threshold or the second threshold, one or more additional microneedles may then be used to sense or measure glucose levels.

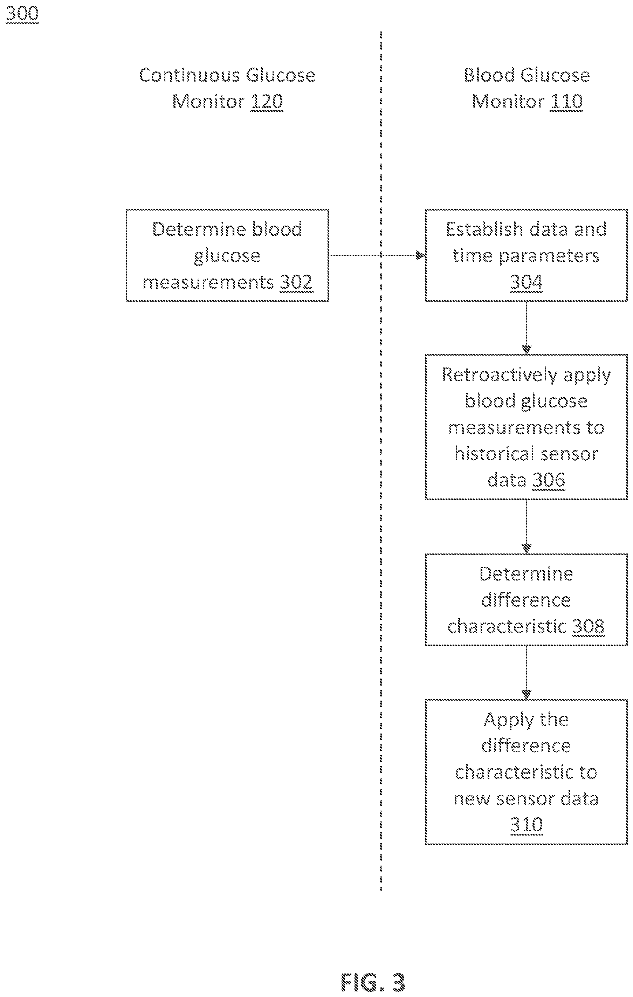

FIG. 3 depicts an example of a data exchange sequence 300 directed to calibration and diagnostic aspects based on data exchange between the blood glucose monitor 110 and the continuous glucose monitor 120.

At 302, the blood glucose monitor 110 determines blood glucose measurements based on one or more blood samples from the user. The blood glucose measurements may be a collection of measurements from one or more days, each associated with a date and time. The blood glucose monitor 110 provides the blood glucose measurements to the continuous glucose monitor 120 to be used in a retrospective calibration process.

At 304, in response to receiving the blood glucose measurements, the continuous glucose monitor 120 establishes date and time parameters based on the date and time associated with each of the blood glucose measurements.

At 306, the continuous glucose monitor 120 retroactively applies the blood glucose measurements to historical sensor data obtained by the continuous glucose monitor 120.

At 308, the continuous glucose monitor 120 determines a difference characteristic. The difference characteristic may be a function or representation that defines a difference between the sensor data monitored by the continuous glucose monitor 120 and the blood glucose measurements determined by the blood glucose monitor 110.

At 310, the continuous glucose monitor 120 applies the difference characteristic to new sensor data and/or updates the algorithm to reflect the difference characteristic. In particular, with the knowledge obtained from historical data from the blood glucose monitor 110, the algorithm implemented by the continuous glucose monitor 120 is adapted to account for measurement discrepancies.

According to some implementations, the blood glucose monitor 110 and the continuous glucose monitor 120 may be integrated into a co-existing structure. By integrating the two traditionally separate devices, the ease of use from the perspective of the user may be increased. The integration of the two separate devices may also reduce usage of materials and provide additional benefits.

In one variation, an applicator housing is configured to securely contain therein the continuous glucose monitor 120. The applicator housing may, together with a cap or cover, provide a sterile and convenient environment for the continuous glucose monitor 120 until application on the user. The applicator housing may include features that engage the continuous glucose monitor 120 within the applicator housing until removal of the cap and activation of an application mechanism. The activation of the application mechanism releases the continuous glucose monitor 120 from the applicator housing for application to the user.

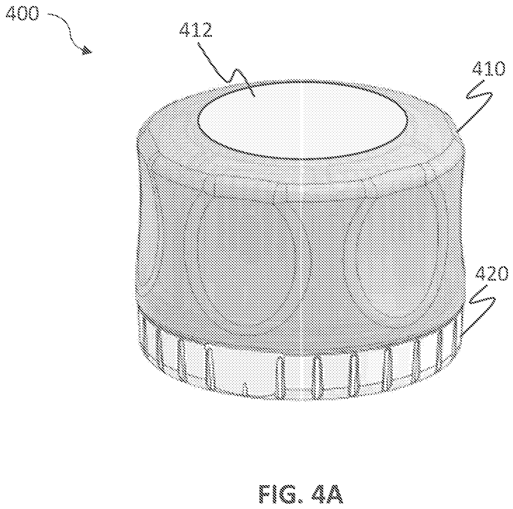

FIG. 4A depicts an illustrative schematic of an example device 400 that incorporates the blood glucose monitor 110 and the continuous glucose monitor 120. The continuous glucose monitor 120 is contained within an enclosure created by the connection of an applicator housing 410 and a removable base 420. The blood glucose monitor 110 is integrated into the applicator housing 410, or, in other variation, the blood glucose monitor 110 is integrated into the removable base 420.

When the removable base 420 is removed and an application mechanism is activated, the continuous glucose monitor 120 is applied to the user. In some variations, the removable base 420 may be reconnected to the applicator housing 410, which internally includes the components of the blood glucose monitor 110, for example, the components described with reference to FIG. 1. In certain variations, the blood glucose monitor contains an embedded port for disposable fingerstick blood test strips. In other variations, the blood glucose monitor contains an embedded lancing device to perform the fingerstick operation to elicit a blood sample. In other variations, the removable base 420 may contain a wireless radio to interface with a continuous glucose monitor device, a wearable device, a smartwatch, and/or a smartphone. A display may or may not be included in the blood glucose monitor.

A display 412 may be provided on an outer surface of the applicator housing 410 and may provide a user interface for the blood glucose monitor 110 according to aspects disclosed herein. In other variations, the removable base 420 may provide a user interface for the blood glucose monitor 110 according to aspects disclosed herein.

With continued reference to FIG. 4A, in some variations, the blood glucose monitor 110 is integrated into the removable base 420. When the removable base 420 is removed and an application mechanism is activated, the continuous glucose monitor 120 is applied to the user. In some variations, the removable base 420 includes the components of the blood glucose monitor 110, for example, the components described with reference to FIG. 1. A display on an outer surface of the removable base 420 may provide a user interface for the blood glucose monitor 110 according to aspects disclosed herein. In certain variations, the blood glucose monitor contains an embedded port for disposable fingerstick blood test strips. In other variations, the blood glucose monitor contains an embedded lancing device to perform the fingerstick operation to elicit a blood sample. In other variations, the removable base 420 may contain a wireless radio to interface with a continuous glucose monitor device, a wearable device, a smartwatch, and/or a smartphone. A display may or may not be included in the blood glucose monitor.

In yet other variations, the continuous glucose monitor 120 may be inserted into the blood glucose monitor 110 integrated into the removable base 420 to enable fingerstick blood glucose monitoring over a prescribed duration until the user wears another continuous glucose monitor 120. In this fashion, the readout circuitry is contained in the continuous glucose monitor 120, alleviating cost and complexity requirements for the blood glucose monitor 110 since the blood glucose monitor device, in this scenario, would not contain an electrochemical analog front end (e.g., potentiostat) and, optionally, the microcontroller. In some variations, the blood glucose monitor contains an embedded power source (e.g., battery) to further sustain or otherwise augment the battery life of the continuous glucose monitor 120 since the continuous glucose monitor 120 is likely to provide sufficient power for a very limited amount of time (e.g., 5 days, 7 days, 10 days, 14 days) owing to its diminutive size.

In other variations, the continuous glucose monitor 120 may continue to illuminate onboard LEDs to indicate the user's glycemic status (e.g., blue for euglycemia, amber for hyperglycemia, purple for hypoglycemia) while embedded in the blood glucose monitor 110 integrated into the removable base 420.

In another variation, the continuous glucose monitor 120 integrated in the blood glucose monitor 110 integrated into the removable base 420 may be configured to be worn or transported by the user to collect kinesthetic (e.g., activity, steps), electrophysiological (e.g., heart rate), opto-physiological (e.g., oxygen saturation), and/or environmental information (e.g., ambient temperature, skin temperature, relative humidity, barometric pressure, ambient light levels, acoustic signatures, gas sensor).

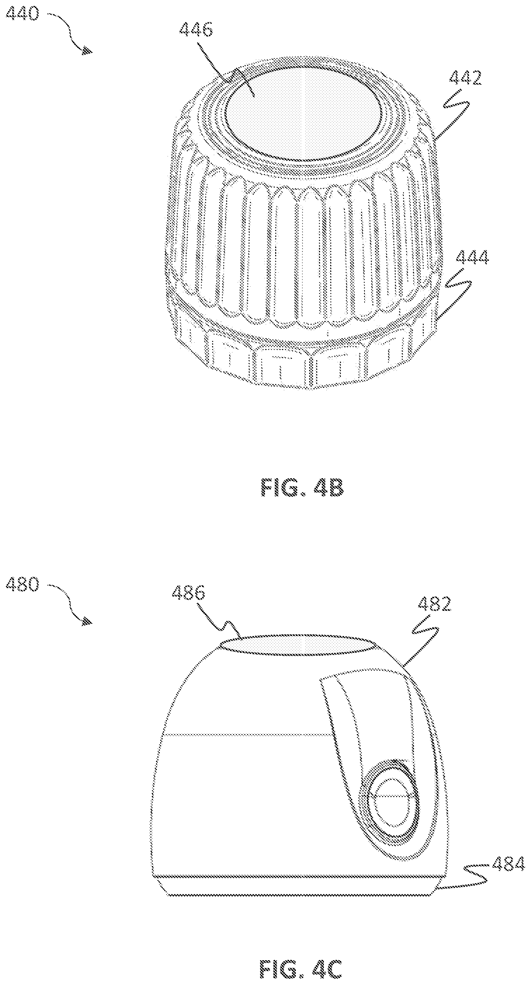

The shape and form of the blood glucose monitor 110 integrated within an applicator housing of the continuous glucose monitor 120 may take various configurations and is not limited to the exact form depicted in FIG. 4A. For example, example devices 440 and 480, as shown in FIG. 4B and FIG. 4C, respectively, or variations thereof may include features of the blood glucose monitor 110 integrated within an applicator housing of the continuous glucose monitor 120. The example devices 440 and 480 include similar components, such as applicator housings 442 and 482, removable bases 444 and 484, and displays 446 and 486, respectively.

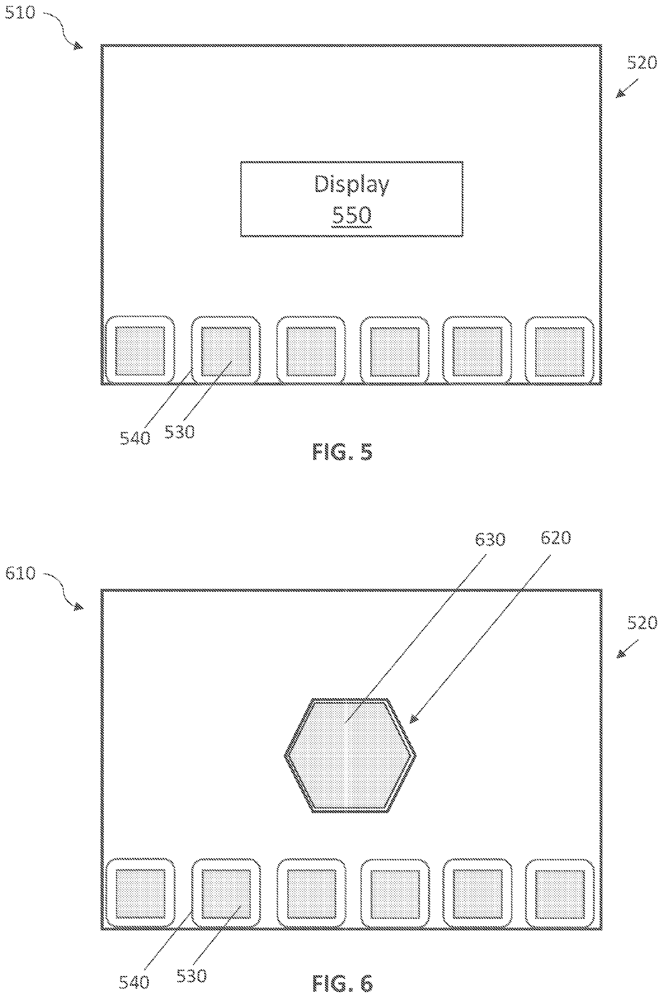

FIG. 5 depicts an illustrative schematic of a multi-day blood glucose monitor 510. The multi-day blood glucose monitor 510 includes a plate 520 on which a plurality of test strip modules 530 are formed on a first surface, which may have a rectangular profile. The plate 520 may define a cavity (e.g., an interior region) such that components may be contained within the cavity, as further described herein. Each test strip module 530 may have a corresponding cover 540 that removably attaches to the test strip module 530. Each test strip module 530 may be an indented or recessed area. In some variations, when the covers 540 are connected to respective ones of the test strip modules 530, the covers 540 may form a flat or substantially flat surface with the first surface of the plate 520. In some variations, a single cover is provided that encases the entirety of the first surface of the plate 520. Any suitable number of test strip modules 530 may be incorporated in the multi-day blood glucose monitor 510.

Each test strip module 530 may include a test strip with suitable reagents that react with a blood sample. A user may take a fingerstick to obtain a blood sample, which may be applied to one of the test strip modules 530 for analysis to measure glucose level in the blood sample. When a user wishes to measure a glucose level, the cover 540 is removed, a fingerstick is taken, and the blood sample is applied to the accessible test strip module 530. The cover 540 may then be reapplied. A display or LED array 550 may be provided in a central area of the plate 520 for outputting the measured glucose level and other outputs according to aspects of the current subject matter described herein. An electrochemical analog front end (e.g., potentiostat), microcontroller, a communication module, a battery, and embedded circuitry may be contained within the interior region of the plate 520. For example, a cavity may be defined within sidewalls of the plate 520. While a rectangular form factor is shown, implementations are not so limited and the plate 520 may be a variety of shapes and sizes. Additionally, the test strip modules 530 and the covers 540 may be of a variety of shapes and sizes.

In other variations, the user may take a fingerstick to obtain a blood sample, which may be applied directly to one of the test strip modules 530 for analysis to measure glucose level in the blood sample; a cover need not be removed nor re-applied to perform the analysis.

In yet other variations, a lancing device may be integrated into the multi-day blood glucose monitor 510.

In yet other variations, the multi-day blood glucose monitor 510 is configured without a display and is intended to relay blood glucose measurements wirelessly to the user's smartphone or smartwatch or other device.

In yet other embodiments, the multi-day blood glucose monitor 510 is packaged along with the applicator housing 410. In other configurations, the multi-day blood glucose monitor 510 is integrated into the applicator cap 420.

FIG. 6 depicts an illustrative schematic of a multi-day blood glucose monitor 810 that incorporates the plate 520, the test strip modules 530, and the covers 540. The multi-day blood glucose monitor 810 also includes a wearable receiver 820 into which a continuous glucose monitor 630 may be securely received and connected. The continuous glucose monitor 630 may be similar or equivalent to the continuous glucose monitor 120 described herein. The continuous glucose monitor 630 has a user interface that displays data representative of measured glucose levels.

In some variations, the continuous glucose monitor 630 may have a limited duration of use, such as between five and 10 days. After use of the continuous glucose monitor 630, the user may measure glucose levels using the test strip modules 530. The incorporation of the continuous glucose monitor 630 into the plate 520 provides for the analytic and user interface capabilities of the continuous glucose monitor 630 to continue to be accessible. For example, in some variations, the wearable receiver 820 includes a connection component for communicatively coupling to the continuous glucose monitor 630, thereby enabling communication and data exchange between the multi-day blood glucose monitor 810 and the continuous glucose monitor 630.

In yet other variations, the continuous glucose monitor 630 may be inserted into the wearable receiver 820 to enable fingerstick blood glucose monitoring over a prescribed duration until the user wears another continuous glucose monitor 630. In this fashion, the readout circuitry is contained in the continuous glucose monitor 630, alleviating cost and complexity requirements for the multi-day blood glucose monitor 810 since the blood glucose monitor device, in this scenario, would not contain the electrochemical analog front end (e.g., potentiostat) and, optionally, the microcontroller. In certain embodiments, the blood glucose monitor would contain an embedded power source (e.g., battery) to further sustain or otherwise augment the battery life of the continuous glucose monitor 630 since the continuous glucose monitor is likely to provide sufficient power for a very limited amount of time (e.g., 5 days, 7 days, 10 days, 14 days) owing to its diminutive size.

In other variations, the continuous glucose monitor 630 may continue to illuminate onboard LEDs to indicate the user's glycemic status (e.g., blue for euglycemia, amber for hyperglycemia, purple for hypoglycemia) while embedded in the receiver 820.

In another variation, the continuous glucose monitor 630 integrated in the receiver 820 may be configured to be worn or transported by the user to collect kinesthetic (e.g., activity, steps), electrophysiological (e.g., heart rate), opto-physiological (e.g., oxygen saturation), and/or environmental information (e.g., ambient temperature, skin temperature, relative humidity, barometric pressure, ambient light levels, acoustic signatures, gas sensor).

The shape and form of the continuous glucose monitor 630 integrated within the wearable receiver 820 may take various configurations and is not limited to the exact form depicted in FIG. 6. Likewise, the shape and form of the multi-day blood glucose monitor 810 may take various configurations and is not limited to the exact form depicted in FIG. 6.

The following provides a description of some examples of a continuous analyte monitor in which the current subject matter may be implemented. Also provided are examples of a microneedle array and microneedle structure for use in the exemplary continuous analyte monitor. The following descriptions are meant to be exemplary, and aspects related to the interconnection and data exchange between a continuous glucose monitor and a blood glucose monitor consistent with the current subject matter are not limited to the exemplary continuous analyte monitor, the exemplary microneedle array, and the exemplary microneedle structure described herein.

As shown in FIG. 7, in some variations, an analyte monitoring device 710 may generally include a housing 712 and a microneedle array 740 extending outwardly from the housing. The housing 712, may, for example, be a wearable housing configured to be worn on the skin of a user such that the microneedle array 740 extends at least partially into the skin of the user. For example, the housing 712 may include an adhesive such that the analyte monitoring device 710 is a skin-adhered patch that is simple and straightforward for application to a user. The microneedle array 740 may be configured to puncture the skin of the user and include one or more electrochemical sensors (e.g., electrodes) configured for measuring one or more target analytes, such as glucose, that are accessible after the microneedle array 740 punctures the skin of the user. In some variations, the analyte monitoring device 710 may be integrated or self-contained as a single unit, and the unit may be disposable (e.g., used for a period of time and replaced with another instance of the analyte monitoring device 710).

An electronics system 720 may be at least partially arranged in the housing 712 and include various electronic components, such as sensor circuitry 724 configured to perform signal processing (e.g., biasing and readout of electrochemical sensors, converting the analog signals from the electrochemical sensors to digital signals, etc.). The electronics system 720 may also include at least one microcontroller 722 for controlling the analyte monitoring device 710, at least one communication module 726, at least one power source 730, and/or other various suitable passive circuitry 727. The microcontroller 722 may, for example, be configured to interpret digital signals output from the sensor circuitry 724 (e.g., by executing a programmed routine in firmware), perform various suitable algorithms or mathematical transformations (e.g., calibration, etc.), and/or route processed data to and/or from the communication module 724. In some variations, the communication module 726 may include a suitable wireless transceiver (e.g., Bluetooth transceiver or the like) for communicating data with an external computing device via one or more antennas 728. For example, the communication module 726 may be configured to provide uni-directional and/or bi-directional communication of data with an external computing device that is paired with the glucose monitoring device 710. The power source 730 may provide power for the analyte monitoring device 710, such as for the electronics system. The power source 730 may include battery or other suitable source, and may, in some variations, be rechargeable and/or replaceable. Passive circuitry 727 may include various non-powered electrical circuitry (e.g., resistors, capacitors, inductors, etc.) providing interconnections between other electronic components, etc. The passive circuitry 727 may be configured to perform noise reduction, biasing and/or other purposes, for example. In some variations, the electronic components in the electronics system 720 may be arranged on one or more printed circuit boards (PCB), which may be rigid, semi-rigid, or flexible, for example.

In some variations, the analyte monitoring device 710 may further include one or more additional sensors 750 to provide additional information that may be relevant for user monitoring. For example, the analyte monitoring device 710 may further include at least one temperature sensor (e.g., thermistor) configured to measure skin temperature, thereby enabling temperature compensation for the sensor measurements obtained by the microneedle array electrochemical sensors.

In some variations, the microneedle array 740 in the analyte monitoring device 710 may be configured to puncture skin of a user. When the analyte monitoring device 710 is worn by the user, the microneedle array 740 may extend into the skin of the user such that electrodes on distal regions of the microneedles rest in the dermis. Specifically, in some variations, the microneedles may be designed to penetrate the skin and access the upper dermal region (e.g., papillary dermis and upper reticular dermis layers) of the skin, in order to enable the electrodes to access interstitial fluid that surrounds the cells in these layers. For example, in some variations, the microneedles may have a height generally ranging between at least 350 µm and about 515 µm. In some variations, one or more microneedles may extend from the housing such that a distal end of the electrode on the microneedle is located less than about 5 mm from a skin-interfacing surface of the housing, less than about 4 mm from the housing, less than about 3 mm from the housing, less than about 2 mm from the housing, or less than about 1 mm from the housing.

In contrast to traditional continuous analyte monitoring devices, which include sensors typically implanted between about 8 mm and about 10 mm beneath the skin surface in the subcutis or adipose layer of the skin, the analyte monitoring device 710 has a shallower microneedle insertion depth of about 0.25 mm (such that electrodes are implanted in the upper dermal region of the skin) that provides numerous benefits. These benefits include access to dermal interstitial fluid including one or more target analytes for detection, which is advantageous at least because at least some types of analyte measurements of dermal interstitial fluid have been found to closely correlate to those of blood. For example, it has been discovered that glucose measurements performed using electrochemical sensors accessing dermal interstitial fluid are advantageously highly linearly correlated with blood glucose measurements. Accordingly, glucose measurements based on dermal interstitial fluid are highly representative of blood glucose measurements.

Additionally, because of the shallower microneedle insertion depth of the analyte monitoring device 710, a reduced time delay in glucose detection is obtained compared to traditional continuous glucose monitoring devices. Such a shallower insertion depth positions the sensor surfaces in close proximity (e.g., within a few hundred micrometers or less) to the dense and well-perfused capillary bed of the reticular dermis, resulting in a negligible diffusional lag from the capillaries to the sensor surface. Diffusion time is related to diffusion distance according to t = x2/(2D) where t is the diffusion time, x is the diffusion distance, and D is the mass diffusivity of the analyte of interest. Therefore, positioning a glucose sensing element twice as far away from the source of glucose in a capillary will result in a quadrupling of the diffusional delay time. Accordingly, conventional glucose sensors, which reside in the very poorly vascularized adipose tissue beneath the dermis, result in a significantly greater diffusion distance from the vasculature in the dermis and thus a substantial diffusional latency (e.g., typically 5 - 20 minutes). In contrast, the shallower microneedle insertion depth of the analyte monitoring device 710 benefits from low diffusional latency from capillaries to the sensor, thereby reducing time delay in analyte detection and providing more accurate results in real-time or near real-time. For example, in some embodiments, diffusional latency may be less than 10 minutes, less than 5 minutes, or less than 3 minutes.

Furthermore, when the microneedle array rests in the upper dermal region, the lower dermis beneath the microneedle array includes very high levels of vascularization and perfusion to support the dermal metabolism, which enables thermoregulation (via vasoconstriction and/or vasodilation) and provides a barrier function to help stabilize the sensing environment around the microneedles. Yet another advantage of the shallower insertion depth is that the upper dermal layers lack pain receptors, thus resulting in a reduced pain sensation when the microneedle array punctures the skin of the user, and providing for a more comfortable, minimally-invasive user experience.

Thus, the analyte monitoring devices and methods described herein enable improved continuous monitoring of one or more target analytes of a user. For example, as described above, the analyte monitoring device may be simple and straightforward to apply, which improves ease-of-use and user compliance. Additionally, glucose measurements of dermal interstitial fluid may provide for highly accurate detection. Furthermore, compared to traditional continuous glucose monitoring devices, insertion of the microneedle array and its sensors may be less invasive and involve less pain for the user.

In some variations, the electronics system of the analyte monitoring device may include an analog front end. The analog front end may include sensor circuitry (e.g., sensor circuitry 724 as shown in FIG. 7) that converts analog current measurements to digital values that can be processed by the microcontroller. The analog front end may, for example, include a programmable analog front end that is suitable for use with electrochemical sensors. For example, the analog front end may include a MAX30131, MAX30132, or MAX30134 component (which have 1, 2, and 4 channel, respectively), available from Maxim Integrated (San Jose, CA), which are ultra-low power programmable analog front ends for use with electrochemical sensors. The analog front end may also include an AD5940 or AD5941 component, available from Analog Devices (Norwood, MA), which are high precision, impedance and electrochemical front ends. Similarly, the analog front end may also include an LMP91000, available from Texas Instruments (Dallas, TX), which is a configurable analog front end potentiostat for low-power chemical sensing applications. The analog front end may provide biasing and a complete measurement path, including the analog to digital converters (ADCs). Ultra-low power may allow for the continuous biasing of the sensor to maintain accuracy and fast response when measurement is required for an extended duration (e.g., seven days) using a body-worn, battery-operated device.

In some variations, the analog front end device may be compatible with both two and three terminal electrochemical sensors, such as to enable both DC current measurement, AC current measurement, and electrochemical impedance spectroscopy (EIS) measurement capabilities. Furthermore, the analog front end may include an internal temperature sensor and programmable voltage reference, support external temperature monitoring and an external reference source and integrate voltage monitoring of bias and supply voltages for safety and compliance.

In some variations, the analog front end may include a multi-channel potentiostat to multiplex sensor inputs and handle multiple signal channels. For example, the analog front end may include a multi-channel potentiostat such as that described in U.S. Patent No. 9,933,387.

In some variations, the analog front end and peripheral electronics may be integrated into an application-specific integrated circuit (ASIC), which may help reduce cost, for example. This integrated solution may include the microcontroller described below, in some variations.

In some variations, the electronics system of the analyte monitoring device may include at least one microcontroller (e.g., controller 722 as shown in FIG. 7). The microcontroller may include, for example, a processor with integrated flash memory. In some variations, the microcontroller in the analyte monitoring device may be configured to perform analysis to correlate sensor signals to an analyte measurement (e.g., glucose measurement). For example, the microcontroller may execute a programmed routine in firmware to interpret the digital signal (e.g., from the analog front end), perform any relevant algorithms and/or other analysis, and route processed data to and/or from the communication module. Keeping the analysis on-board the analyte monitoring device may, for example, enable the analyte monitoring device to broadcast analyte measurement(s) to multiple devices (e.g., mobile computing devices such as a smartphone or smartwatch, therapeutic delivery systems such as insulin pens or pumps, etc.) in parallel, while ensuring that each connected device has the same information.

In some variations, the microcontroller may be configured to activate and/or inactivate the analyte monitoring device on one or more detected conditions. For example, the device may be configured to power on the analyte monitoring device upon insertion of the microneedle array into skin. This may, for example, enable a power-saving feature in which the battery is disconnected until the microneedle array is placed in skin, at which time the device may begin broadcasting sensor data. Such a feature may, for example, help improve the shelf life of the analyte monitoring device and/or simplify the analyte monitoring device-external device pairing process for the user.

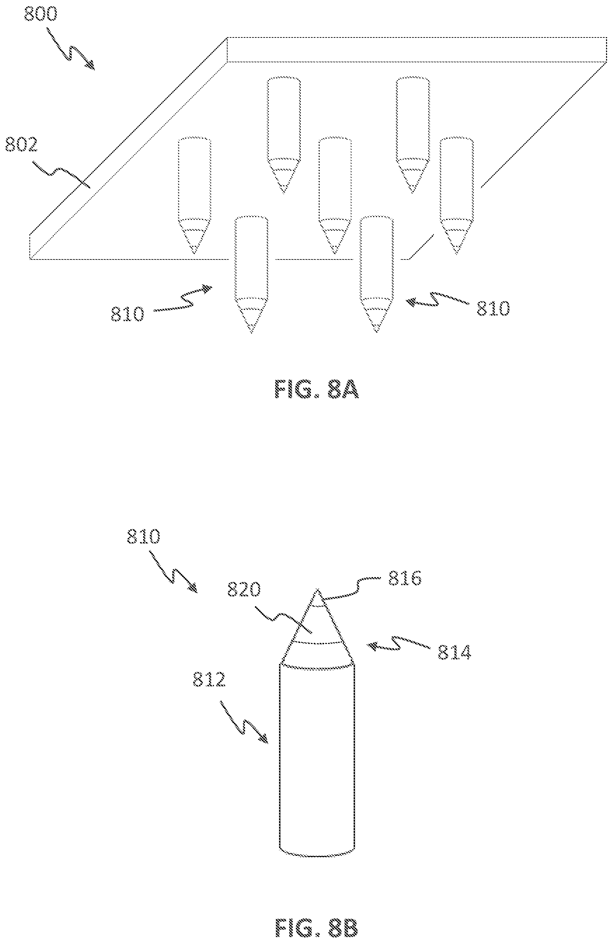

As shown in the schematic of FIG. 8A, in some variations, a microneedle array 800 for use in sensing one or more analytes may include one or more microneedles 810 projecting from a substrate surface 802. The substrate surface 802 may, for example, be generally planar and one or more microneedles 810 may project orthogonally from the planar surface. Generally, as shown in FIG. 8B, a microneedle 810 may include a body portion 812 (e.g., shaft) and a tapered distal portion 814 configured to puncture skin of a user. In some variations, the tapered distal portion 814 may terminate in an insulated distal apex 816. The microneedle 810 may further include an electrode 820 on a surface of the tapered distal portion. In some variations, electrode-based measurements may be performed at the interface of the electrode and interstitial fluid located within the body (e.g., on an outer surface of the overall microneedle). In some variations, the microneedle 810 may have a solid core (e.g., solid body portion), though in some variations the microneedle 810 may include one or more lumens, which may be used for drug delivery or sampling of the dermal interstitial fluid, for example. Other microneedle variations, such as those described below, may similarly either include a solid core or one or more lumens.

The microneedle array 800 may be at least partially formed from a semiconductor (e.g., silicon) substrate and include various material layers applied and shaped using various suitable microelectromechanical systems (MEMS) manufacturing techniques (e.g., deposition and etching techniques), as further described below. The microneedle array may be reflow-soldered to a circuit board, similar to a typical integrated circuit. Furthermore, in some variations the microneedle array 800 may include a three electrode setup including a working (sensing) electrode having an electrochemical sensing coating (including a biorecognition element such as an enzyme) that enables detection of a target analyte, a reference electrode, and a counter electrode. In other words, the microneedle array 800 may include at least one microneedle 810 that includes a working electrode, at least one microneedle 810 including a reference electrode, and at least one microneedle 810 including a counter electrode. Additional details of these types of electrodes are described in further detail below.

In some variations, the microneedle array 800 may include a plurality of microneedles that are insulated such that the electrode on each microneedle in the plurality of microneedles is individually addressable and electrically isolated from every other electrode on the microneedle array. The resulting individual addressability of the microneedle array 800 may enable greater control over each electrode's function, since each electrode may be separately probed. For example, the microneedle array 800 may be used to provide multiple independent measurements of a given target analyte, which improves the device's sensing reliability and accuracy. Furthermore, in some variations the electrodes of multiple microneedles may be electrically connected to produce augmented signal levels. As another example, the same microneedle array 800 may additionally or alternatively be interrogated to simultaneously measure multiple analytes to provide a more comprehensive assessment of physiological status. For example, a microneedle array may include a portion of microneedles to detect a first Analyte A, a second portion of microneedles to detect a second Analyte B, and a third portion of microneedles to detect a third Analyte C. It should be understood that the microneedle array may be configured to detect any suitable number of analytes (e.g., 1, 2, 3, 4, 5 or more, etc.). Suitable target analytes for detection may, for example, include glucose, ketones, lactate, and cortisol. Thus, individual electrical addressability of the microneedle array 800 provides greater control and flexibility over the sensing function of the analyte monitoring device.

In some variations of microneedles (e.g., microneedles with a working electrode), the electrode 820 may be located proximal to the insulated distal apex 816 of the microneedle. In other words, in some variations the electrode 820 does not cover the apex of the microneedle. Rather, the electrode 820 may be offset from the apex or tip of the microneedle. The electrode 820 being proximal to or offset from the insulated distal apex 816 of the microneedle advantageously provides more accurate sensor measurements. For example, this arrangement prevents concentration of the electric field at the microneedle apex 816 during manufacturing, thereby avoiding non-uniform electro-deposition of sensing chemistry on the surface of the electrode 820 that would result in faulty sensing.

As another example, placing the electrode 820 offset from the microneedle apex further improves sensing accuracy by reducing undesirable signal artefacts and/or erroneous sensor readings caused by stress upon microneedle insertion. The distal apex of the microneedle is the first region to penetrate into the skin, and thus experiences the most stress caused by the mechanical shear phenomena accompanying the tearing or cutting of the skin. If the electrode 820 were placed on the apex or tip of the microneedle, this mechanical stress may delaminate the electrochemical sensing coating on the electrode surface when the microneedle is inserted, and/or cause a small yet interfering amount of tissue to be transported onto the active sensing portion of the electrode. Thus, placing the electrode 820 sufficiently offset from the microneedle apex may improve sensing accuracy. For example, in some variations, a distal edge of the electrode 820 may be located at least about 10 µm (e.g., between about 20 µm and about 30 µm) from the distal apex or tip of the microneedle, as measured along a longitudinal axis of the microneedle.

The body portion 812 of the microneedle 810 may further include an electrically conductive pathway extending between the electrode 820 and a backside electrode or other electrical contact (e.g., arranged on a backside of the substrate of the microneedle array). The backside electrode may be soldered to a circuit board, enabling electrical communication with the electrode 820 via the conductive pathway. For example, during use, the in-vivo sensing current (inside the dermis) measured at a working electrode is interrogated by the backside electrical contact, and the electrical connection between the backside electrical contact and the working electrode is facilitated by the conductive pathway. In some variations, this conductive pathway may be facilitated by a metal via running through the interior of the microneedle body portion (e.g., shaft) between the microneedle's proximal and distal ends. Alternatively, in some variations the conductive pathway may be provided by the entire body portion being formed of a conductive material (e.g., doped silicon). In some of these variations, the complete substrate on which the microneedle array 800 is built upon may be electrically conductive, and each microneedle 810 in the microneedle array 800 may be electrically isolated from adjacent microneedles 810 as described below. For example, in some variations, each microneedle 810 in the microneedle array 800 may be electrically isolated from adjacent microneedles 810 with an insulative barrier including electrically insulative material (e.g., dielectric material such as silicon dioxide) that surrounds the conductive pathway extending between the electrode 820 and backside electrical contact. For example, body portion 812 may include an insulative material that forms a sheath around the conductive pathway, thereby preventing electrical communication between the conductive pathway and the substrate. Other example variations of structures enabling electrical isolation among microneedles are described in further detail below.

Such electrical isolation among microneedles in the microneedle array permits the sensors to be individually addressable. This individually addressability advantageously enables independent and parallelized measurement among the sensors, as well as dynamic reconfiguration of sensor assignment (e.g., to different analytes). In some variations, the electrodes in the microneedle array can be configured to provide redundant analyte measurements, which is an advantage over conventional analyte monitoring devices. For example, redundancy can improve performance by improving accuracy (e.g., averaging multiple analyte measurement values for the same analyte which reduces the effect of extreme high or low sensor signals on the determination of analyte levels) and/or improving reliability of the device by reducing the likelihood of total failure.

In some variations, the microneedle array may be formed at least in part with suitable semiconductor and/or MEMS fabrication techniques and/or mechanical cutting or dicing. Such processes may, for example, be advantageous for enabling large-scale, cost-efficient manufacturing of microneedle arrays.

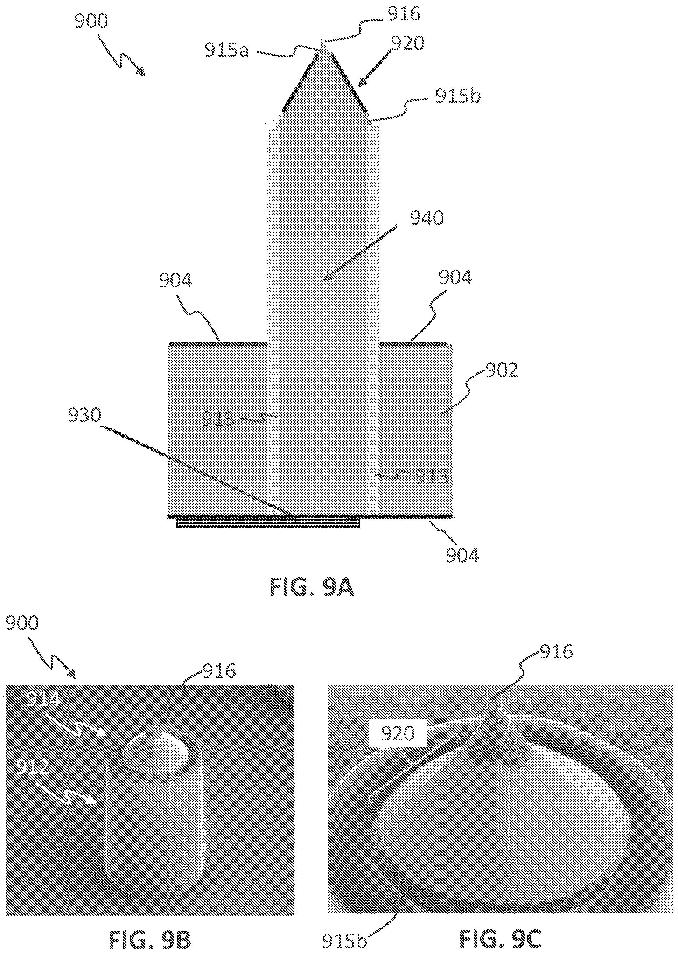

In some variations, a microneedle may have a generally columnar body portion and a tapered distal portion with an electrode. For example, FIGS. 9A-9C illustrate an example variation of a microneedle 900 extending from a substrate 902. FIG. 9A is a side cross-sectional view of a schematic of microneedle 900, while FIG. 9B is a perspective view of the microneedle 900 and FIG. 9C is a detailed perspective view of a distal portion of the microneedle 900. As shown in FIGS. 9B and 9C, the microneedle 900 may include a columnar body portion 912, a tapered distal portion 914 terminating in an insulated distal apex 916, and an annular electrode 920 that includes a conductive material (e.g., Pt, Ir, Au, Ti, Cr, Ni, etc.) and is arranged on the tapered distal portion 914. As shown in FIG. 9A, the annular electrode 920 may be proximal to (or offset or spaced apart from) the distal apex 916. For example, the electrode 920 may be electrically isolated from the distal apex 916 by a distal insulating surface 915a including an insulating material (e.g., SiO₂). In some variations, the electrode 920 may also be electrically isolated from the columnar body portion 912 by a second distal insulating surface 915b. The electrode 920 may be in electrical communication with a conductive core 940 (e.g., conductive pathway) passing along the body portion 912 to a backside electrical contact 930 (e.g., made of Ni/Au alloy) or other electrical pad in or on the substrate 902. For example, the body portion 912 may include a conductive core material (e.g., highly doped silicon). As shown in FIG. 9A, in some variations, an insulating moat 913 including an insulating material (e.g., SiO₂) may be arranged around (e.g., around the perimeter) of the body portion 912 and extend at least partially through the substrate 902. Accordingly, the insulating moat 913 may, for example, help prevent electrical contact between the conductive core 940 and the surrounding substrate 902. The insulating moat 913 may further extend over the surface of the body portion 912. Upper and/or lower surfaces of the substrate 902 may also include a layer of substrate insulation 904 (e.g., SiO₂). Accordingly, the insulation provided by the insulating moat 913 and/or substrate insulation 904 may contribute at least in part to the electrical isolation of the microneedle 900 that enables individual addressability of the microneedle 900 within a microneedle array. Furthermore, in some variations the insulating moat 913 extending over the surface of the body portion 912 may function to increase the mechanical strength of the microneedle 900 structure.

The microneedle 900 may be formed at least in part by suitable MEMS fabrication techniques such as plasma etching, also called dry etching. For example, in some variations, the insulating moat 913 around the body portion 912 of the microneedle may be made by first forming a trench in a silicon substrate by deep reactive ion etching (DRIE) from the backside of the substrate, then filling that trench with a sandwich structure of SiO₂ / polycrystalline silicon (poly-Si) / SiO₂ by low pressure chemical vapor deposition (LPCVD) or other suitable process. In other words, the insulating moat 913 may passivate the surface of the body portion 912 of the microneedle and continue as a buried feature in the substrate 902 near the proximal portion of the microneedle. By including largely compounds of silicon, the insulating moat 913 may provide good fill and adhesion to the adjoining silicon walls (e.g., of the conductive core 940, substrate 902, etc.). The sandwich structure of the insulating moat 913 may further help provide excellent matching of coefficient of thermal expansion (CTE) with the adjacent silicon, thereby advantageously reducing faults, cracks, and/or other thermally-induced weaknesses in the insulating moat 913.

The tapered distal portion may be fashioned out by an isotropic dry etch from the frontside of the substrate, and the body portion 912 of the microneedle 900 may be formed from DRIE. The frontside metal electrode 920 may be deposited and patterned on the distal portion by specialized lithography (e.g., electron-beam evaporation) that permits metal deposition in the desired annular region for the electrode 920 without coating the distal apex 916. Furthermore, the backside electrical contact 930 of Ni/Au may be deposited by suitable MEMS manufacturing techniques (e.g., sputtering).

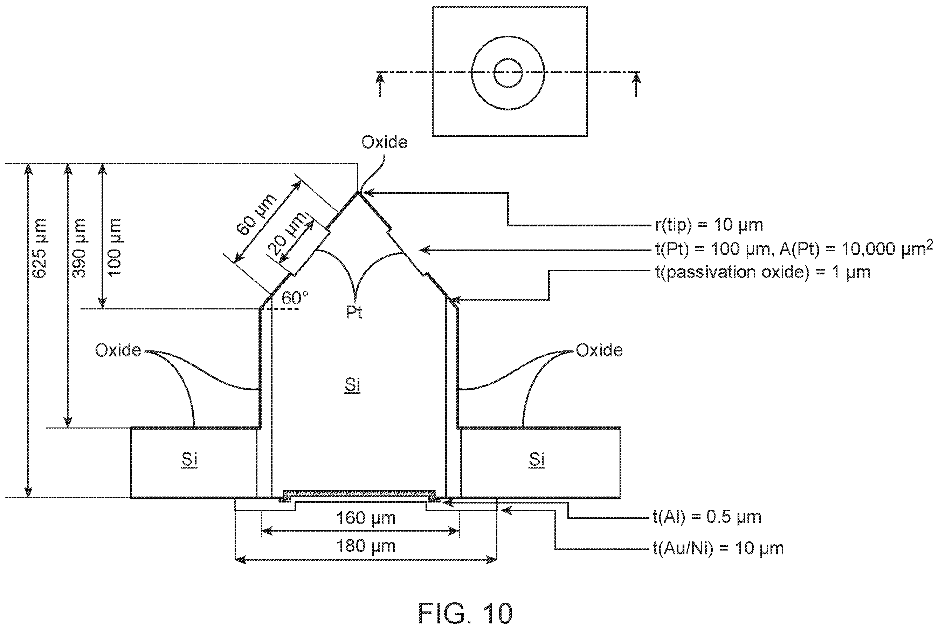

The microneedle 900 may have any suitable dimensions. By way of illustration, the microneedle 900 may, in some variations, have a height of between about 300 µm and about 500 µm. In some variations, the tapered distal portion 914 may have a tip angle between about 60 degrees and about 80 degrees, and an apex diameter of between about 1 µm and about 15 µm. In some variations, the surface area of the annular electrode 920 may include between about 9,000 µm² and about 11,000 µm², or about 10,000 µm². FIG. 10 illustrates various dimensions of an example variation of a columnar microneedle with a tapered distal portion and annular electrode, similar to microneedle 900 described above.

The microneedle structure used with variations of the current subject matter is not limited to the structure described above. Microneedles having various forms and characteristics may be used with the continuous glucose monitor and blood glucose monitor data exchange architecture described herein.

Each microneedle in the microneedle array may include an electrode. In some variations, multiple distinct types of electrodes may be included among the microneedles in the microneedle array. For example, in some variations the microneedle array may function as an electrochemical cell operable in an electrolytic manner with three types of electrodes. In other words, the microneedle array may include at least one working electrode, at least one counter electrode, and at least one reference electrode. Thus, the microneedle array may include three distinct electrode types, though one or more of each electrode type may form a complete system (e.g., the system might include multiple distinct working electrodes). Furthermore, multiple distinct microneedles may be electrically joined to form an effective electrode type (e.g., a single working electrode may be formed from two or more connected microneedles with working electrode sites). Each of these electrode types may include a metallization layer and may include one or more coatings or layers over the metallization layer that help facilitate the function of that electrode.

Generally, the working electrode is the electrode at which oxidation and/or reduction reaction of interest occurs for detection of an analyte of interest. The counter electrode functions to source (provide) or sink (accumulate) the electrons, via an electrical current, that are required to sustain the electrochemical reaction at the working electrode. The reference electrode functions to provide a reference potential for the system; that is, the electrical potential at which the working electrode is biased is referenced to the reference electrode. A fixed, time-varying, or at least controlled potential relationship is established between the working and reference electrodes, and within practical limits no current is sourced from or sinked to the reference electrode. Additionally, to implement such a three-electrode system, the analyte monitoring device may include a suitable potentiostat or electrochemical analog front end to maintain a fixed potential relationship between the working electrode and reference electrode contingents within the electrochemical system (via an electronic feedback mechanism), while permitting the counter electrode to dynamically swing to potentials required to sustain the redox reaction of interest.

Multiple microneedles (e.g., any of the microneedle variations described herein, each of which may have a working electrode, counter electrode, or reference electrode as described above) may be arranged in a microneedle array. Considerations of how to configure the microneedles include factors such as desired insertion force for penetrating skin with the microneedle array, optimization of electrode signal levels and other performance aspects, manufacturing costs and complexity, etc.

For example, the microneedle array may include multiple microneedles that are spaced apart at a predefined pitch (distance between the center of one microneedle to the center of its nearest neighboring microneedle). In some variations, the microneedles may be spaced apart with a sufficient pitch so as to distribute force (e.g., avoid a "bed of nails" effect) that is applied to the skin of the user to cause the microneedle array to penetrate the skin. As pitch increases, force required to insert the microneedle array tends to decrease and depth of penetration tends to increase. However, it has been found that pitch only begins to affect insertion force at low values (e.g., less than about 150 µm). Accordingly, in some variations the microneedles in a microneedle array may have a pitch of at least 200 µm, at least 300 µm, at least 400 µm, at least 500 µm, at least 600 µm, at least 700 µm, or at least 750 µm. For example, the pitch may be between about 200 µm and about 800 µm, between about 300 µm and about 700 µm, or between about 400 µm and about 600 µm. In some variations, the microneedles may be arranged in a periodic grid, and the pitch may be uniform in all directions and across all regions of the microneedle array. Alternatively, the pitch may be different as measured along different axes (e.g., X, Y directions) and/or some regions of the microneedle array may include a smaller pitch while other may include a larger pitch.

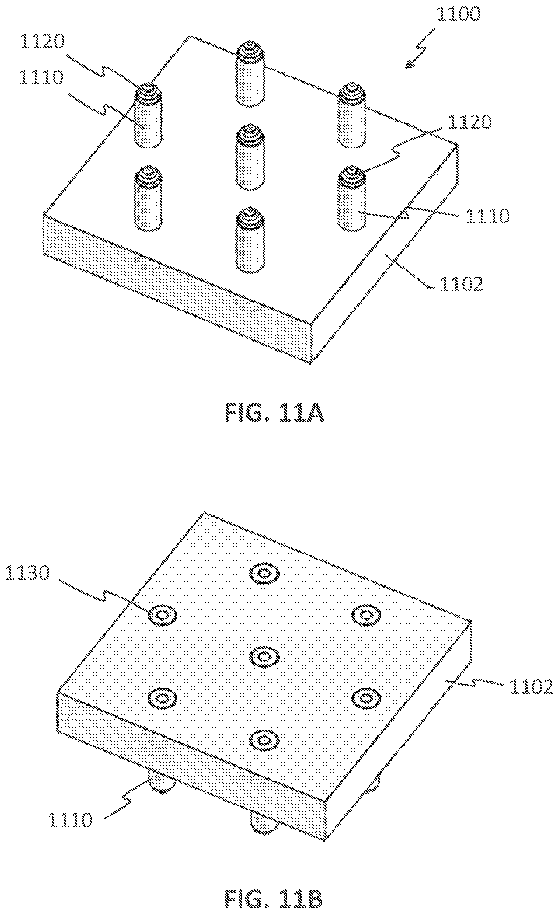

Furthermore, for more consistent penetration, microneedles may be spaced equidistant from one another (e.g., same pitch in all directions). To that end, in some variations, the microneedles in a microneedle array may be arranged in a hexagonal configuration as shown in FIGS. 11A-11C. Alternatively, the microneedles in a microneedle array may arranged in a rectangular array (e.g., square array), or in another suitable symmetrical manner

Another consideration for determining configuration of a microneedle array is overall signal level provided by the microneedles. Generally, signal level at each microneedle is invariant of the total number of microneedle elements in an array. However, signal levels can be enhanced by electrically interconnecting multiple microneedles together in an array. For example, an array with a large number of electrically connected microneedles is expected to produce a greater signal intensity (and hence increased accuracy) than one with fewer microneedles. However, a higher number of microneedles on a die will increase die cost (given a constant pitch) and will also require greater force and/or velocity to insert into skin. In contrast, a lower number of microneedles on a die may reduce die cost and enable insertion into the skin with reduced application force and/or velocity. Furthermore, in some variations a lower number of microneedles on a die may reduce the overall footprint area of the die, which may lead to less unwanted localized edema and/or erythema. Accordingly, in some variations, a balance among these factors may be achieved with a microneedle array including, for example, a microneedle array including seven microneedles as shown in FIGS. 11A - 11C. However, in other variations there may be fewer microneedles or more microneedles in an array (e.g., between about 5 and about 37, between about 5 and about 30, between about 5 and about 25, between about 5 and about 20, between about 5 and about 15, between about 5 and about 100, between about 10 and about 30, between about 15 and about 25, etc.) or more microneedles in an array (e.g., more than 37, more than 40, more than 45, etc.).

Additionally, as described in further detail below, in some variations only a subset of the microneedles in a microneedle array may be active during operation of the analyte monitoring device. For example, a portion of the microneedles in a microneedle array may be inactive (e.g., no signals read from electrodes of inactive microneedles). In some variations, a portion of the microneedles in a microneedle array may be activated at a certain time during operation and remain active for the remainder of the operating lifetime of the device. Furthermore, in some variations, a portion of the microneedles in a microneedle array may additionally or alternatively be deactivated at a certain time during operation and remain inactive for the remainder of the operating lifetime of the device.

In considering characteristics of a die for a microneedle array, die size is a function of the number of microneedles in the microneedle array and the pitch of the microneedles. Manufacturing costs are also a consideration, as a smaller die size will contribute to lower cost since the number of dies that can be formed from a single wafer of a given area will increase. Furthermore, a smaller die size will also be less susceptible to brittle fracture due to the relative fragility of the substrate.

Furthermore, in some variations, microneedles at the periphery of the microneedle array (e.g., near the edge or boundary of the die, near the edge or boundary of the housing, near the edge or boundary of an adhesive layer on the housing, along the outer border of the microneedle array, etc.) may be found to have better performance (e.g., sensitivity) due to better penetration compared to microneedles in the center of the microneedle array or die. Accordingly, in some variations, working electrodes may be arranged largely or entirely on microneedles located at the periphery of the microneedle array, to obtain more accurate and/or precise analyte measurements.

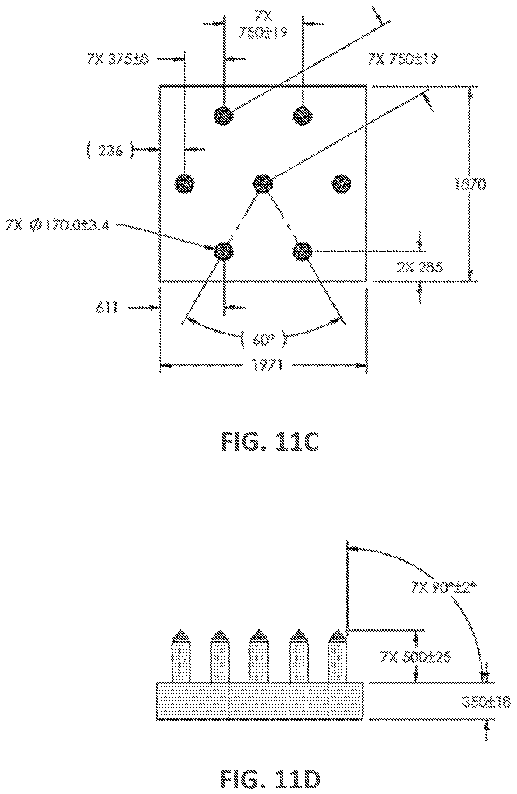

FIGS. 11A and 11B depict perspective views of an illustrative schematic of seven microneedles 1110 arranged in an example variation of a microneedle array 1100. The seven microneedles 1110 are arranged in a hexagonal array on a substrate 1102. As shown in FIG. 11A , the electrodes 1120 are arranged on distal portions of the microneedles 1110 extending from a first surface of the substrate 1102. As shown in FIG. 11B, proximal portions of the microneedles 1110 are conductively connected to respective backside electrical contacts 1130 on a second surface of the substrate 1102 opposite the first surface of the substrate 1102. FIGS. 11C and 11D depict plan and side views of an illustrative schematic of a microneedle array similar to microneedle array 1100. As shown in FIGS. 11C and 11D, the seven microneedles are arranged in a hexagonal array with an inter-needle center-to-center pitch of about 750 µm between the center of each microneedle and the center of its immediate neighbor in any direction. In other variations the inter-needle center-to-center pitch may be, for example, between about 700 µm and about 800 µm, or between about 725 µm and about 775 µm. The microneedles may have an approximate outer shaft diameter of about 170 µm (or between about 150 µm and about 190 µm, or between about 125 µm and about 200 µm) and a height of about 500 µm (or between about 475 µm and about 525 µm, or between about 450 µm and about 550 µm).

Furthermore, the microneedle arrays described herein may have a high degree of configurability concerning where the working electrode(s), counter electrode(s), and reference electrode(s) are located within the microneedle array. This configurability may be facilitated by the electronics system.

As described herein, in some variations the analyte monitoring device 110 may be applied using a suitable applicator. The applicator may, for example, be configured to urge the analyte monitoring device 110 toward the skin of the user such that the microneedle array 140 is inserted into the skin (e.g., to the desired target depth) and the one or more adhesive layers are adhered to the skin to securely hold the analyte monitoring device 110 in place.

While it is generally understood that the invention described herein relates to glucose monitoring, the disclosed concepts may be applied broadly to analyte monitoring, which may include, but is not limited to, lactate monitoring, ketone monitoring, cortisol monitoring, monitoring of other metabolites, monitoring hormones, or monitoring of neurotransmitters.

The foregoing description, for purposes of explanation, uses specific nomenclature to provide a thorough understanding of the invention. However, it will be apparent to one skilled in the art that specific details are not required to practice the invention. Thus, the foregoing descriptions of specific embodiments of the invention are presented for purposes of illustration and description. They are not intended to be exhaustive or to limit the invention to the precise forms disclosed; obviously, many modifications and variations are possible in view of the above teachings. The embodiments were chosen and described to explain the principles of the invention and its practical applications; they thereby enable others skilled in the art to utilize the invention and various embodiments with various modifications as are suited to the particular use contemplated. The following claims define the scope of the invention.

## Claims

1. A system (100), comprising:
a microneedle array (740, 800, 1100) comprising a plurality of microneedles (810, 1110), wherein at least a first microneedle (810, 900, 1110) and a second microneedle (810, 900, 1110) of the plurality of microneedles (810) are configured to sense glucose levels in dermal interstitial fluid of a user; and
one or more processors and at least one memory storing instructions which, when executed by the one or more processors, result in operations comprising:
determining that a first difference between a first glucose level measured by the first microneedle (810, 900, 1110) and a second glucose level measured by the second microneedle (810, 900, 1110) exceeds a first threshold;
transmitting to a blood glucose monitor (110, 510, 810) a request to receive a glucose measurement;
determining, in response to the glucose measurement received from the blood glucose monitor (110, 510, 810), that a second difference between the first glucose level measured by the first microneedle and the glucose measurement from the blood glucose monitor (110, 510, 810) exceeds a second threshold; and
discarding, in response to determining that the second difference exceeds the second threshold, the first glucose level measured by the first microneedle (810, 900, 1110) in a resultant glucose level outputted on a user interface (114, 124).

2. The system of claim 1, wherein the first threshold and the second threshold are equal.

3. The system of claim 1, wherein the first threshold and/or the second threshold are user-defined and/or user-adjusted parameters.

4. The system of claim 1, wherein each of the first microneedle and the second microneedle comprise a working electrode.

5. The system of claim 4, wherein the plurality of microneedles further comprises a counter electrode and a reference electrode.

6. The system of claim 1, wherein the operations further comprise:
incorporating the first glucose level measured by the first microneedle in the resultant glucose level in response to determining that the second difference no longer exceeds the second threshold.

7. The system of claim 6, wherein the operations further comprise:
in response to a determination that a threshold period of time from which the second difference exceeds the second threshold has elapsed, discontinuing use of the first microneedle.

8. The system of claim 1, wherein the request transmitted to the blood glucose monitor is in the form of one or more wireless signals.

## Patentansprüche

1. System (100), umfassend:
eine Mikronadelanordnung (740, 800, 1100), die eine Mehrzahl von Mikronadeln (810, 1110) umfasst, wobei wenigstens eine erste Mikronadel (810, 900, 1110) und eine zweite Mikronadel (810, 900, 1110) der Mehrzahl von Mikronadeln (810) dazu ausgelegt sind, Glukosespiegel in dermaler interstitieller Flüssigkeit eines Benutzers zu erfassen; und
einen oder mehrere Prozessoren und wenigstens einen Speicher, der Anweisungen speichert, die bei Ausführung durch die ein oder mehreren Prozessoren Operationen zur Folge haben, die Folgendes umfassen:
Bestimmen, dass eine erste Differenz zwischen einem ersten Glukosespiegel, der von der ersten Mikronadel (810, 900, 1110) gemessen wird, und einem zweiten Glukosespiegel, der von der zweiten Mikronadel (810, 900, 1110) gemessen wird, einen ersten Schwellwert überschreitet;
Übertragen, an einen Blutzuckermonitor (110, 510, 810), einer Anforderung zum Empfangen einer Glukosemessung;
Bestimmen, in Reaktion auf die Glukosemessung, die von dem Blutzuckermonitor (110, 510, 810) empfangen wird, dass eine zweite Differenz zwischen dem ersten Glukosespiegel, der von der ersten Mikronadel gemessen wird, und der Glukosemessung von dem Blutzuckermonitor (110, 510, 810) einen zweiten Schwellwert überschreitet; und
Verwerfen, in Reaktion auf das Bestimmen, dass die zweite Differenz den zweiten Schwellwert überschreitet, des ersten Glukosespiegels, der von der ersten Mikronadel (810, 900, 1110) gemessen wird, in einem resultierenden Glukosespiegel, der über eine Benutzerschnittstelle (114, 124) ausgegeben wird.

2. System nach Anspruch 1, wobei der erste Schwellwert und der zweite Schwellwert gleich sind.

3. System nach Anspruch 1, wobei der erste Schwellwert und/und der zweite Schwellwert benutzerdefinierte und/oder von einem Benutzer angepasste Parameter sind.

4. System nach Anspruch 1, wobei die erste Mikronadel und die zweite Mikronadel jeweils eine Arbeitselektrode umfassen.

5. System nach Anspruch 4, wobei die Mehrzahl von Mikronadeln ferner eine Gegenelektrode und eine Referenzelektrode umfasst.

6. System nach Anspruch 1, wobei die Operationen ferner umfassen:
Integrieren des ersten Glukosespiegels, der von der ersten Mikronadel gemessen wird, in den resultierenden Glukosespiegel, in Reaktion auf das Bestimmen, dass die zweite Differenz den zweiten Schwellwert nicht mehr überschreitet.

7. System nach Anspruch 6, wobei die Operationen ferner umfassen:
in Reaktion auf eine Bestimmung, dass ein Schwellwertzeitraum, in dem die zweite Differenz den zweiten Schwellwert überschreitet, verstrichen ist, Aussetzen der Verwendung der ersten Mikronadel.

8. System nach Anspruch 1, wobei die Anforderung, die an den Blutzuckermonitor übertragen wird, in Form eines oder mehrerer Drahtlossignale vorliegt.

## Revendications

1. Système (100) comprenant :
un réseau de micro-aiguilles (740, 800, 1100) comprenant une pluralité de micro-aiguilles (810, 1110), au moins une première micro-aiguille (810, 900, 1110) et une seconde micro-aiguille (810, 900, 1110) de la pluralité de micro-aiguilles (810) étant configurées pour détecter les niveaux de glucose dans le fluide interstitiel dermique d'un utilisateur ; et
un ou plusieurs processeurs et au moins une mémoire mémorisant des instructions qui, lorsqu'elles sont exécutées par le ou les processeurs, entraînent des opérations comprenant :
la détermination qu'une première différence entre un premier niveau de glucose mesuré par la première micro-aiguille (810, 900, 1110) et un second niveau de glucose mesuré par la seconde micro-aiguille (810, 900, 1110) dépasse un premier seuil ;
la transmission à un dispositif de surveillance de glycémie (110, 510, 810) d'une demande de recevoir une mesure de glycémie ;
la détermination, en réponse à la mesure de glucose reçue du dispositif de surveillance de glycémie (110, 510, 810), qu'une seconde différence entre le premier niveau de glucose mesuré par la première micro-aiguille et la mesure de glucose provenant du dispositif de surveillance de glycémie (110, 510, 810) dépasse un second seuil ; et l'élimination, en réponse à la détermination que la seconde différence dépasse le second seuil, du premier niveau de glucose mesuré par la première micro-aiguille (810, 900, 1110) dans un niveau de glucose résultant émis sur une interface utilisateur (114, 124).

2. Système selon la revendication 1, le premier seuil et le second seuil étant égaux.

3. Système selon la revendication 1, le premier seuil et/ou le second seuil étant des paramètres définis par l'utilisateur et/ou ajustés par l'utilisateur.

4. Système selon la revendication 1, chacune de la première micro-aiguille et de la seconde micro-aiguille comprenant une électrode de travail.

5. Système selon la revendication 4, la pluralité de micro-aiguilles comprenant en outre une contre-électrode et une électrode de référence.

6. Système selon la revendication 1, les opérations comprenant en outre :
l'incorporation du premier niveau de glucose mesuré par la première micro-aiguille au niveau de glucose résultant en réponse à la détermination que la seconde différence ne dépasse plus le second seuil.

7. Système selon la revendication 6, les opérations comprenant en outre :
en réponse à la détermination qu'une période de temps seuil à partir de laquelle la seconde différence dépasse le second seuil s'est écoulée, l'utilisation de la première micro-aiguille étant interrompue.

8. Système selon la revendication 1, la demande transmise au dispositif de surveillance de glycémie se présentant sous la forme d'un ou plusieurs signaux sans fil.
